# EUROPEAN PATENT APPLICATION

(11) **EP 1 884 257 A1**
(43) Date of publication of application: **06.02.2008**
(21) Application number: 06254060.4
(22) Date of filing: 02.08.2006
(51) Int. Cl.: A61M 25/06

(54) **Intravenous catheter introducing device with a tubular outlet member**

(71) Applicant: Shue, Ming-Jeng, Hsi Dist, Taichung City (TW); Huang, Deborah, Taichung City (TW); Shue, Phillip, Hsi District, Taichung City (TW)
(72) Inventor: Shue, Ming-Jeng, Hsi Dist, Taichung City (TW); Huang, Deborah, Taichung City (TW); Shue, Phillip, Hsi District, Taichung City (TW)
(74) Representative: Lord, Hilton David

(57) **Abstract**

An intravenous catheter introducing device includes a needle cannula (21) with a rear connecting end (211) that is forced by a needle retracting member (8) to displace relative to a barrel (7) between a position of use to permit a needle tip (212) to pierce a patient's vein, and a disposal position to permit retraction of the needle tip (212) into the barrel (7). A tubular outlet member (30) is disposed downstream of the rear connecting end (211), and is mounted radially and outwardly of the barrel (7) . A flashback member (1, 3, 6, 6' 86) can be connected to and in fluid communication with the tubular outlet member (30) so as to permit the user to check whether a tubular catheter (23) has been successfully introduced into a target vein of the patient. In addition, the flashback member (1,3,6,6') can provide a blood drawing function to facilitate flow of blood into the flashback member (1,3,6,6').

## Description

This invention relates to an intravenous catheter introducing device, more particularly to an intravenous catheter introducing device which has a tubular outlet member that is in fluid communication with a needle cannula and that is disposed outwardly of a barrel so as to be connected to a flashback member for facilitating the catheter introducing operation.

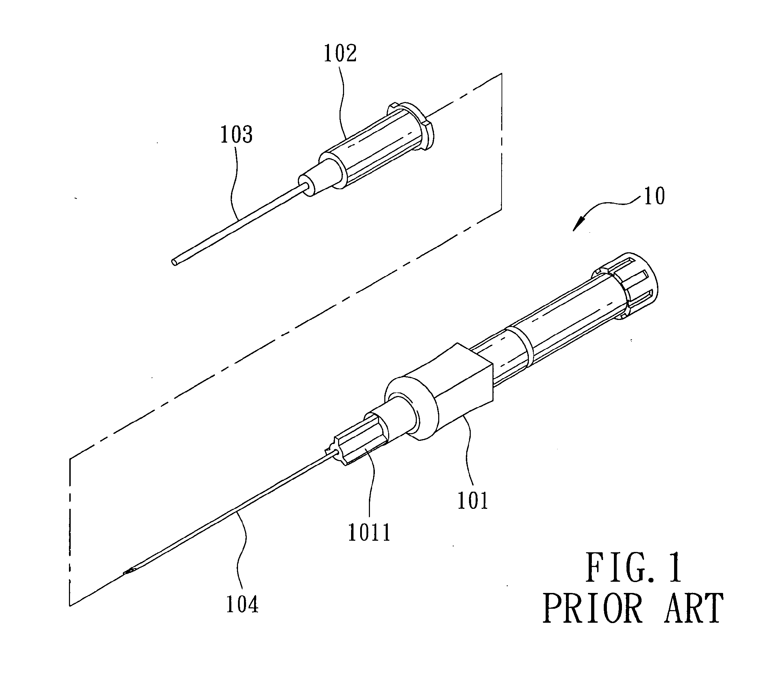

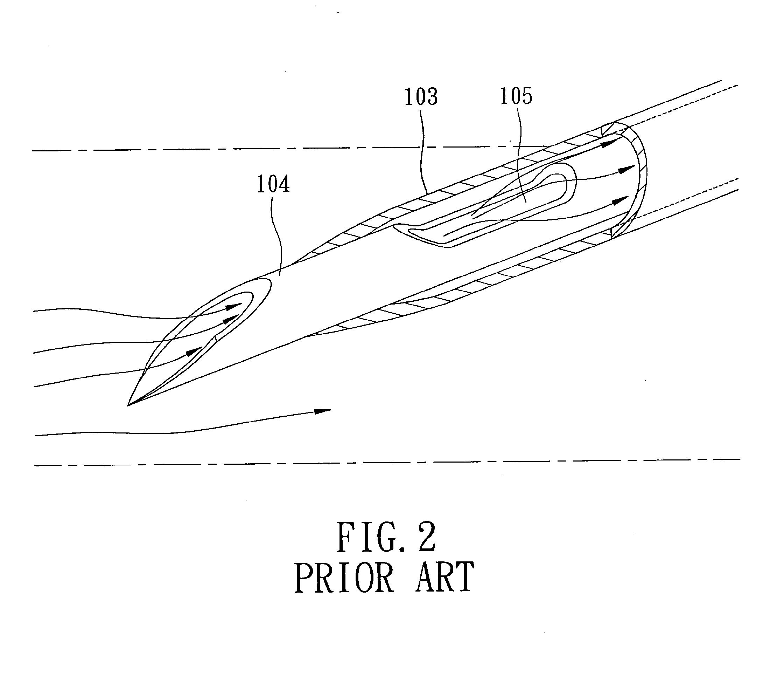

Intravenous catheter inserting devices are generally used to administer medication fluids into or draw blood from a patient's vein. Referring to Figs. 1 and 2, a conventional intravenous catheter inserting device 10 is shown to include a tubular needle seat 101 with a hub end 1011, a needle cannula 104 secured to the hub end 1011, a catheter hub 102 sleeved on the needle seat 1011, and a flexible tubular catheter 103 secured to the catheter hub 102. In use, the catheter 103 and the needle cannula 104 are inserted into the patient' s vein by a health care worker by piercing the patient's vein with a sharp tip of the needle cannula 104 which projects outwardly of the catheter 103. The health care worker then withdraws the needle cannula 104 from the catheter 103 with one hand and, at the same time, applies pressure to the patient's skin with the other hand, thereby leaving the catheter 103 in the patient's vein. Subsequently, a transfusion member (not shown) with a medication fluid or an empty barrel is connected to the catheter hub 102 for administering the medication fluid into the patient's vein or for drawing blood. At this time, as the health care worker must place the used needle cannula 104 and the needle seat 101 on a tray (not shown) nearby, the exposed sharp tip of the used needle cannula 104 may create a danger of an accidental needle stick. Moreover, blood contamination may occur during connection of the catheter hub 103 to the transfusion member or the empty barrel.

Referring to Fig. 2, during the insertion procedure, the flow of blood into the catheter 103 through a notch 105 in the needle cannula 104 can be observed through the translucent catheter 103. However, due to the presence of the notch 105, the needle cannula 104 tends to be bent during passage of the catheter 103 into the patient's vein. In addition, the blood in the catheter 103 is not visible when the portion of the needle cannula 104 with the notch 105 is inserted into the patient's vein.

Moreover, the conventional intravenous catheter inserting device 10 is specifically not suitable for patients whose blood pressure is not sufficient to permit flow of blood through the notch 105, such as an emergency case, aged people, and pediatrics patients, and patients whose target vein is barely visible due to abundant adipose tissue, such as women and obese patients, since the health care worker will have difficulty determining whether the catheter 103 has been successfully introduced into the target vein, and may need to locate the vein by moving the needle cannula 104 in the skin of the patient, thereby complicating and prolonging the cannulation procedure and causing great discomfort to the patient.

The object of the present invention is to provide an intravenous catheter introducing device which has a tubular outlet member that is in fluid communication with a needle cannula and that is disposed outwardly of a barrel so as to be connected to a flashback member for facilitating the catheter introducing operation.

Another object of the present invention is to provide an intravenous catheter introducing device which ensures retraction of a needle cannula into a barrel after use.

According to this invention, the intravenous catheter introducing device includes a needle cannula having a front segment which extends along an axis to terminate at a tip end, and a rear connecting end opposite to the front segment. A tubular outlet member is disposed downstream of the rear connecting end of the needle cannula in terms of inflow of blood sample through the needle cannula and which is radially offset from the axis. A catheter hub defines therein a duct that permits extension of the front segment of the needle cannula therethrough. A tubular catheter has a proximate segment which is inserted into the duct, and a distal segment which extends from the proximate segment to surround and sheathe the front segment of the needle cannula while permitting the tip end to project forwardly of the distal segment for piercing a patient's skin. A barrel has a nose tubular end portion which is detachably coupled with the duct in the catheter hub, and which defines therein a passage that is disposed to be communicated with the duct and that is configured to permit the front segment of the needle cannula to pass therethrough, and a barrel wall which extends from the nose tubular end portion rearwardly along the axis to terminate at a tail end portion such that the tubular outlet member is disposed outwardly of the barrel wall. The barrel wall defines a guiding groove that is interposed between the nose tubular end portion and the tail end portion, and that is communicated with the passage. A needle retracting member is disposed to be slidable relative to the barrel wall toward the tail end portion, and which includes a force effecting end and a shifter. The force effecting end is received in the guiding groove to force the needle cannula to move rearwards when the needle retracting member is moved to slide toward the tail end portion. A shifter is configured to extend radially relative to the axis and outwardly of the barrel wall such that when the shifter is caused to move by a user's fingertip, the needle retracting member is moved to slide from a position of use, where the needle retracting member is closer to the nose tubular end portion, to a disposal position, where the needle retracting member is closer to the tail end portion so as to withdraw the tip end of the needle cannula into the barrel wall.

Other features and advantages of the present invention will become apparent in the following detailed description of the preferred embodiments of the invention, with reference to the accompanying drawings, in which:
Fig. 1 is a perspective view of a conventional intravenous catheter inserting device;
Fig. 2 is a partly sectional schematic view illustrating a needle cannula sleeved over by a catheter of the conventional intravenous catheter inserting device;
Fig. 3 is a sectional exploded view of the first preferred embodiment of an intravenous catheter introducing device according to this invention;
Fig. 4 is a sectional view of the first preferred embodiment;
Fig. 5 is a schematic top view of the first preferred embodiment;
Fig. 6 is a cross-sectional view of a bracing member partly surrounding a barrel of the first preferred embodiment;
Fig. 7 is a sectional view of the first preferred embodiment when a needle cannula is retracted;
Fig. 8 is a sectional view of the first preferred embodiment when a protecting cap is sleeved on a front end of the barrel;
Fig. 9 is a sectional view of the second preferred embodiment of an intravenous catheter introducing device according to this invention;

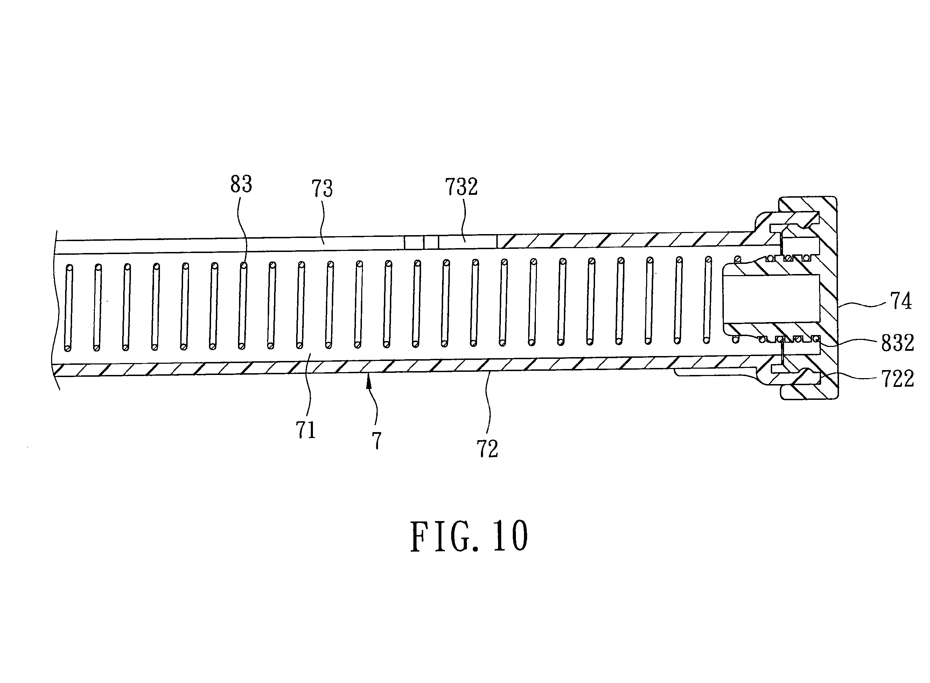

Fig. 10 is a fragmentary sectional view of a modified form of the second preferred embodiment;
Fig. 11 is a schematic top view of the second preferred embodiment;
Fig. 12 is a sectional view of the second preferred embodiment when a needle cannula is retracted;
Fig. 13 is a schematic top view of the third preferred embodiment of an intravenous catheter introducing device according to this invention;
Fig. 14 is a schematic top view of the fourth preferred embodiment of an intravenous catheter introducing device according to this invention;
Fig. 15 is a sectional view of a portion of the fifth preferred embodiment of an intravenous catheter introducing device according to this invention;
Fig. 16 is a sectional view of the sixth preferred embodiment of an intravenous catheter introducing device according to this invention;
Fig. 17 is a sectional view of the sixth preferred embodiment when a needle cannula is retracted;
Fig. 18 is a sectional view of the seventh preferred embodiment of an intravenous catheter introducing device according to this invention;
Fig. 19 is a sectional view of the seventh preferred embodiment when a needle cannula is retracted;
Fig. 20 is a sectional view of the eighth preferred embodiment of an intravenous catheter introducing device according to this invention;
Fig. 21 is a sectional view of a modified form of the eighth preferred embodiment;
Fig. 22 is a sectional view of the ninth preferred embodiment of an intravenous catheter introducing device according to this invention;
Fig. 23 is a sectional view of a portion of the tenth preferred embodiment of an intravenous catheter introducing device according to this invention;
Fig. 24 is a sectional view of the eleventh preferred embodiment of an intravenous catheter introducing device according to this invention;
Fig. 25 is a sectional view of the eleventh preferred embodiment when a needle cannula is retracted;
Fig. 26 is a sectional view of the twelfth preferred embodiment of an intravenous catheter introducing device according to this invention;
Fig. 27 is a sectional view of a modified form of the twelfth preferred embodiment;

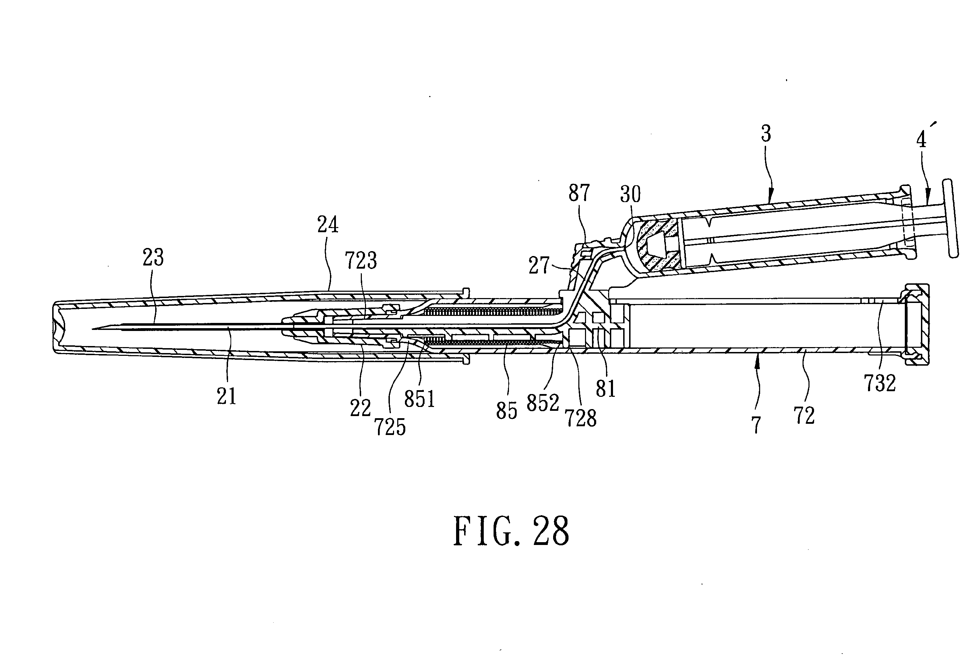

Fig. 28 is a sectional view of the thirteenth preferred embodiment of an intravenous catheter introducing device according to this invention;

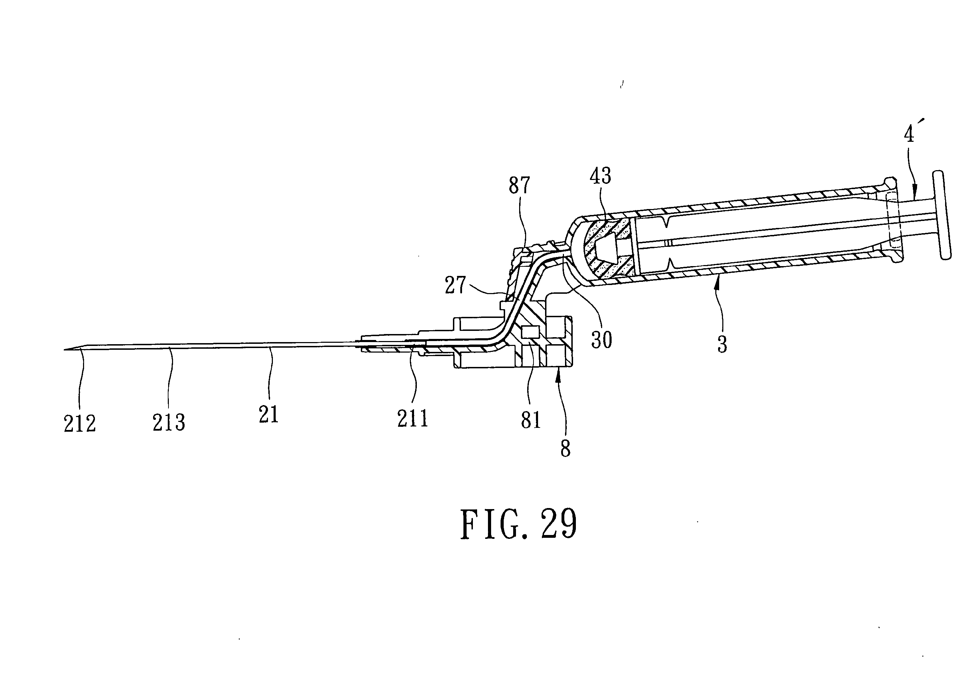

Fig. 29 is a sectional view of a portion of the fourteenth preferred embodiment of an intravenous catheter introducing device according to this invention;
Fig. 30 is a sectional view of the fifteenth preferred embodiment of an intravenous catheter introducing device according to this invention;
Fig. 31 is a sectional view of the fifteenth preferred embodiment when a protecting cap is sleeved on a front end of a barrel;
Fig. 32 is a sectional view of the sixteenth preferred embodiment of an intravenous catheter introducing device according to this invention;
Fig. 33 is a sectional view of a modified form of the sixteenth preferred embodiment;
Fig. 34 is a sectional view of the seventeenth preferred embodiment of an intravenous catheter introducing device according to this invention;

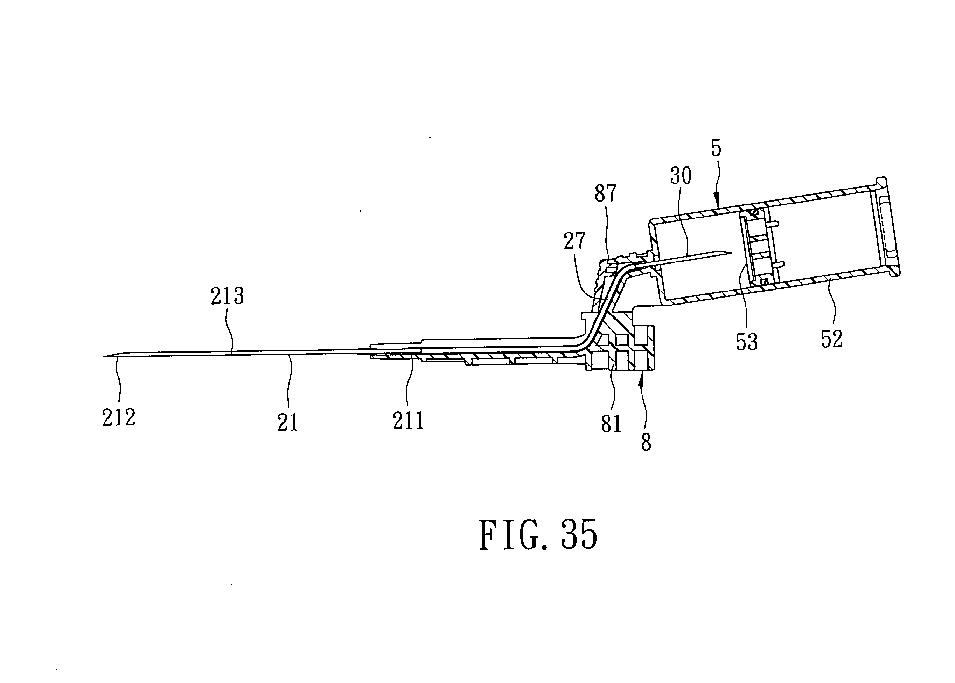

Fig. 35 is a sectional view of a portion of the eighteenth preferred embodiment of an intravenous catheter introducing device according to this invention;
Fig. 36 is a sectional view of the nineteenth preferred embodiment of an intravenous catheter introducing device according to this invention;
Fig. 37 is a sectional view of the twentieth preferred embodiment of an intravenous catheter introducing device according to this invention;
Fig. 38 is a sectional view of a modified form of the twentieth preferred embodiment;
Fig. 39 is a sectional view of the twenty-first preferred embodiment of an intravenous catheter introducing device according to this invention;
Fig. 40 is a sectional view of a portion of the twenty-second preferred embodiment of an intravenous catheter introducing device according to this invention;
Fig. 41 is a sectional view of the twenty-third preferred embodiment of an intravenous catheter introducing device according to this invention;
Fig. 42 is a sectional view of a modified form of the twenty-third preferred embodiment;
Fig. 43 is a sectional view of the twenty-fourthpreferred embodiment of an intravenous catheter introducing device according to this invention;
Fig. 44 is a fragmentary sectional view of the twenty-fourth preferred embodiment;
Fig. 45 is a sectional view of the twenty-fifth preferred embodiment of an intravenous catheter introducing device according to this invention;
Fig. 46 is a sectional view of the twenty-sixth preferred embodiment of an intravenous catheter introducing device according to this invention;
Fig. 47 is a sectional view of the twenty-sixth preferred embodiment when a needle cannula is retracted;
Fig. 48 is a sectional view of the twenty-seventh preferred embodiment of an intravenous catheter introducing device according to this invention;
Fig. 49 is a sectional view of a modified form of the twenty-seventh preferred embodiment;
Fig. 50 is a sectional view of the twenty-eighth preferred embodiment of an intravenous catheter introducing device according to this invention;
Fig. 51 is a sectional view of the twenty-ninth preferred embodiment of an intravenous catheter introducing device according to this invention;
Fig. 52 is a sectional view of the twenty-ninth preferred embodiment when a catheter hub and a tubular catheter are removed;
Fig. 53 is a sectional view of the thirtieth preferred embodiment of an intravenous catheter introducing device according to this invention;
Fig. 54 is a sectional view of a modified form of the thirtieth preferred embodiment; and
Fig. 55 is a sectional view of the thirty-first preferred embodiment of an intravenous catheter introducing device according to this invention.

Before the present invention is described in greater detail, it should be noted that same reference numerals have been used to denote like elements throughout the specification.

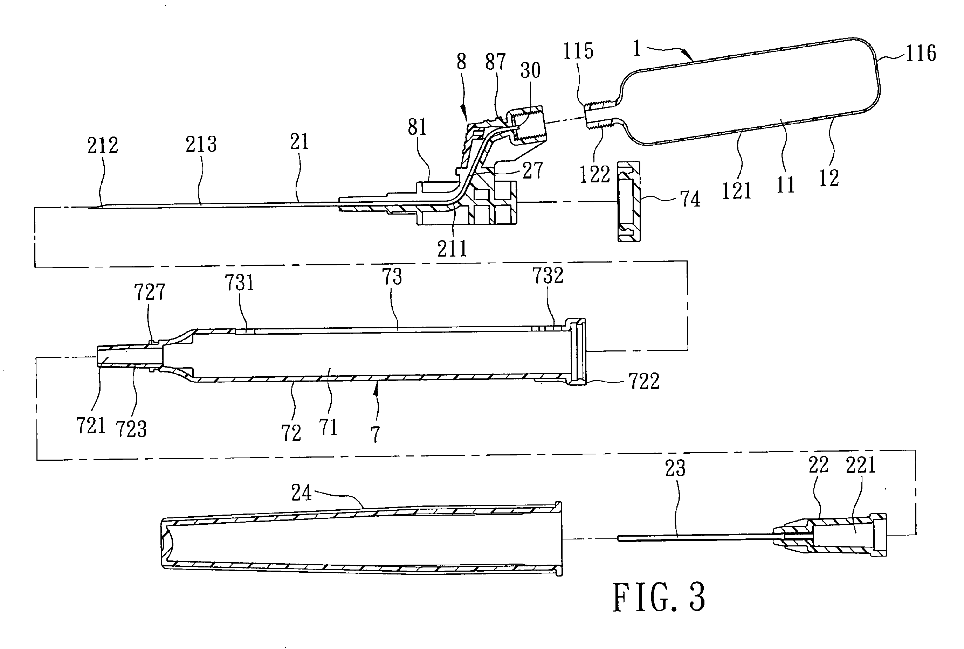

Referring to Figs. 3 to 5, the first preferred embodiment of an intravenous catheter introducing device according to the present invention is shown to comprise a needle cannula 21, a catheter hub 22, a tubular catheter 23, a barrel 7, a needle retracting member 8, a tip protector 24, and a flashback member 1.

The needle cannula 21 has a front segment 213 which extends along an axis (X) and which terminates at a tip end 212, and a rear connecting end 211 opposite to the front segment 213. A tubular bent member 27 is bent relative to and extends from the rear connecting end 211, and terminates at a tubular outlet member 30 such that the tubular outlet member 30 is disposed downstream of the rear connecting end 211 in terms of inflow of blood sample, and is radially offset from the axis (X). In this embodiment, the tubular bent member 27 is integrally formed with the needle cannula 21 and the tubular outlet member 30.

The catheter hub 22 defines a duct 221 therein, which permits extension of the front segment 213 of the needle cannula 21 therethrough. The tubular catheter 23 has a proximate segment which is inserted into the duct 221 of the catheter hub 22, and a distal segment which extends from the proximate segment to surround and sheathe the front segment 213 of the needle cannula 21 while permitting the tip end 212 to project forwardly of the distal segment for piercing a patient's skin.

The barrel 7 has a nose tubular end portion 723 which is detachably inserted into the duct 221 in the catheter hub 22, and which defines therein a passage 721 that is disposed to be communicated with the duct 221 and that is configured to permit the front segment 213 of the needle cannula 21 to pass therethrough, and a barrel wall 72 which extends from the nose tubular end portion 723 rearwardly along the axis (X) to terminate at a tail end portion 722 such that the bent member 27 and the tubular outlet member 30 are disposed outwardly of the barrel wall 72. The barrel wall 72 defines a guiding groove 71 which is interposed between the nose tubular end portion 723 and the tail end portion 722, and is communicated with the passage 721. An end cap 74 is fixedly disposed on the tail end portion 722 to close the guiding groove 71 so as to prevent an undesired enlargement of the tail end portion 722.

The barrel 7 further has an elongated guideway 73 which extends transversely through the barrel wall 72 to be communicated with the guiding groove 71, and which has front and rear retaining regions 731,732 that are proximate to and distal from the nose tubular end portion 723, respectively. In addition, a retaining ring 727 is disposed on the nose tubular end portion 723.

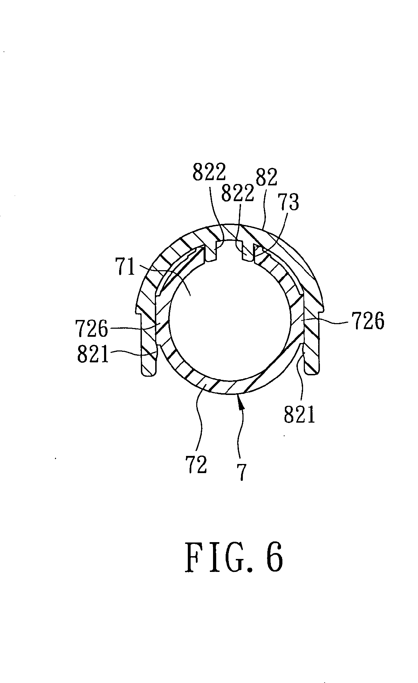

Referring to Fig. 6, the barrel wall 72 further has two side embosses 726 at two sides of the elongated guideway 73.

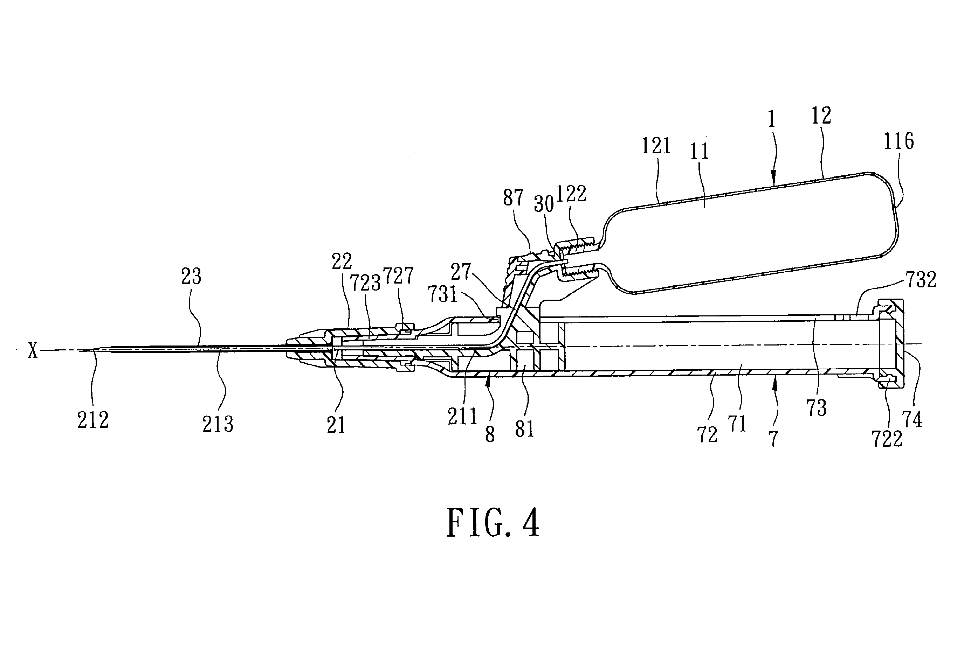

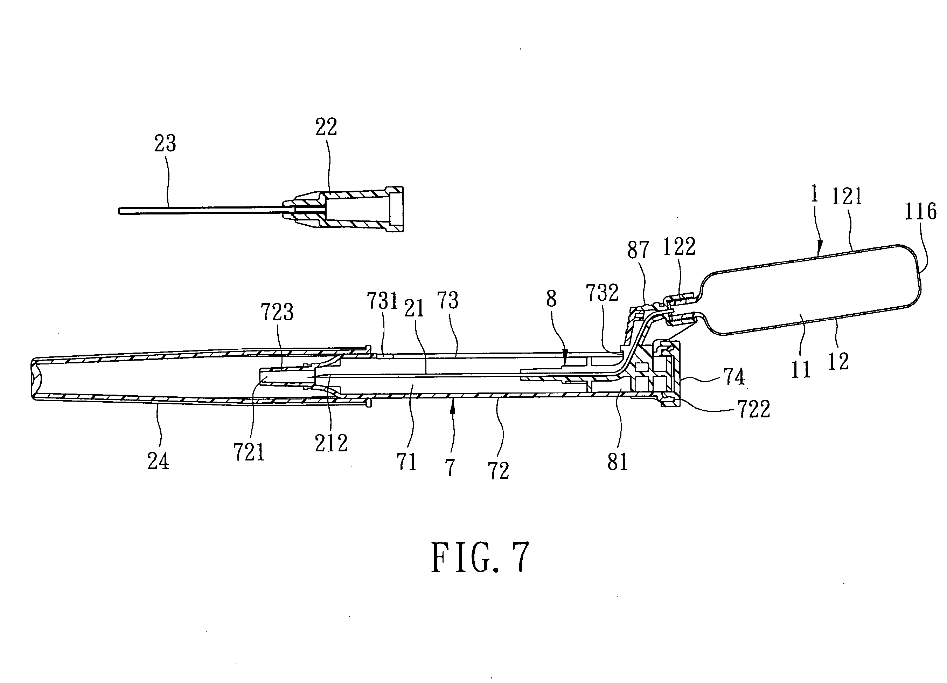

The needle retracting member 8 is disposed to be slidable relative to the barrel wall 72 toward the tail end portion 722, and includes a force effecting end 81 which is received in the guiding groove 71, and which engages the rear connecting end 211 of the needle cannula 21 to force the needle cannula 21 to move rearwards when the needle retracting member 8 is moved to slide toward the tail end portion 722, and a shifter 87 which is configured to extend radially relative to the axis (X) and outwardly of the elongated guideway 73. Thus, when the shifter 87 is caused to slide by a user's fingertip along the elongated guideway 73, the needle retracting member 8 is moved to slide from a position of use, as shown in Fig. 4, where the needle retracting member 8 is closer to the nose tubular end portion 723, to a disposal position, as shown in Fig. 7, where the needle retracting member 8 is closer to the tail end portion 722 so as to withdraw the tip end 212 of the needle cannula 21 into the guiding groove 71. In addition, the shifter 87 is retained in the front and rear retaining regions 731, 732, respectively, when the needle retracting member 8 is displaced to the position of use and the disposal position, respectively.

The tip protector 24 is detachably sleeved on the barrel wall 72 for shielding the needle cannula 21 and the tubular catheter 23.

The flashback member 1 has front and rear ends 115, 116 opposite to each other in a longitudinal direction substantially parallel to the axis (X), and a surrounding wall 12 interconnecting the front and rear ends 115,116. The surrounding wall 12 includes a rear wall portion 121 which is proximate to the rear end 116, and which cooperates with the rear end 116 to define a flashback chamber 11, and a front wall portion 122 which is proximate to the front end 115, and which is disposed to threadedly engage the shifter 87 such that the flashback chamber 11 is disposed downstream of the tubular outlet member 30.

In this embodiment, the surrounding wall 12 of the flashback member 1 is made from a deformable material and is closed by the rear end 116 such that the flashback member 1 can be squeezed by the user to vary the volume of an accommodation space of the flashback chamber 11, thereby generating a reduced pressure in the flashback chamber 11 in a ready position, where the tip end 212 is permitted to search for a vein of a patient for taking a blood sample after the tip end 212 has pierced into the skin of the patient, while a biasing force acting on the flashback chamber 11 is generated as a result of a pressure difference between the reduced pressure and the pressure of an ambient atmosphere.

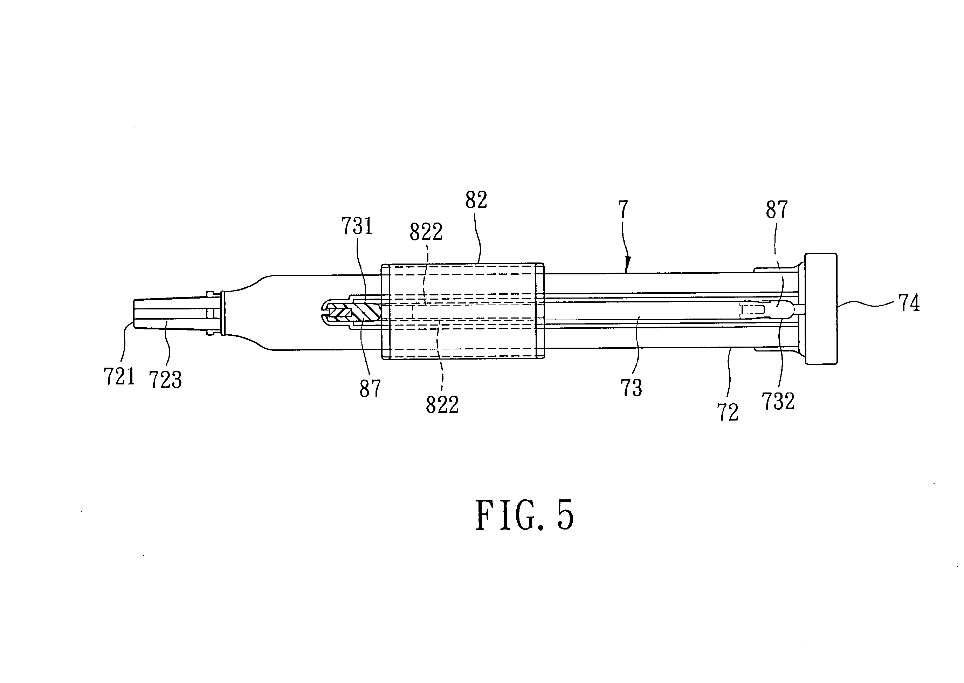

In addition, referring to Figs. 5 and 6, a bracing member 82 has an inner wall surface configured to partly surround the barrel wall 72 about the axis (X) and to cross over the elongated guideway 73 to terminate at two jaw regions 821 that are disposed to abut against the side embosses 726 of the barrel wall 72 at two diametrically opposite areas, and two rib barriers 822 that are configured to be inserted into the elongated guideway 73, and that cooperate with the jaw regions 821 to guard against an undesired enlargement of the elongated guideway 73.

In use, after the tip protector 24 is removed, the user can hold the barrel wall 72 with one hand, and pierce a target vein of the patient with the tip end 212 so that blood flows into the flashback chamber 11 to indicate a successful introduction of the tubular catheter 23. If blood is not observed in the flashback chamber 11, the user has to squeeze the rear wall portion 121 of the flashback member 1 in advance, and then gradually release the rear wall portion 121 to generate an urging force, thereby facilitating flow of blood into the flashback chamber 11. Thus, the user can check whether the tubular catheter 23 has been successfully introduced into the target vein of the patient.

After the catheter hub 22 is removed from the nose tubular end portion 723, the user can press the shifter 87 rearwards with the hand that grips the barrel 7 so as to permit disengagement of the shifter 87 from the front retaining region 731 for releasing the needle retracting member 8 relative to the barrel wall 72. Subsequently, the shifter 87 is moved rearwards along the elongated guideway 73 to the rear retaining region 732 so as to withdraw the tip end 212 of the needle cannula 21 into the guiding groove 71 for subsequent safe disposal. It is noted that during operation, once the tip end 212 has pierced into the skin of the patient, the flashback chamber 11 can be held in the ready position by the biasing force without application of any manual force until the blood flows into the flashback chamber 11 through the needle cannula 21 by virtue of the urging force, i.e., the restoring force of the deformable material of the rear wall portion 121. Thus, the user can conduct the catheter introducing and needle disposal operations according to this invention with a single hand.

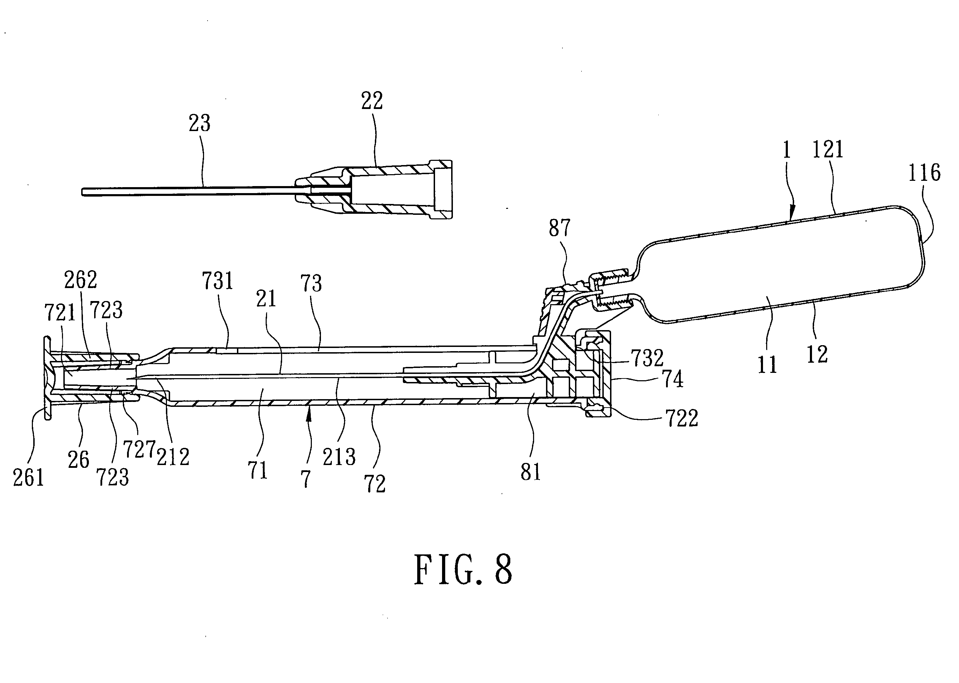

Thereafter, referring to Fig. 8, a protecting cap 26 has a cap surrounding wall 262 which is detachably sleeved on the nose tubular end portion 723 and which is retained by the retaining ring 727, and a cap endwall 261 which is disposed forwardly of the nose tubular end portion 723 so as to close the passage 721 when the needle cannula 21 is withdrawn into the barrel wall 72.

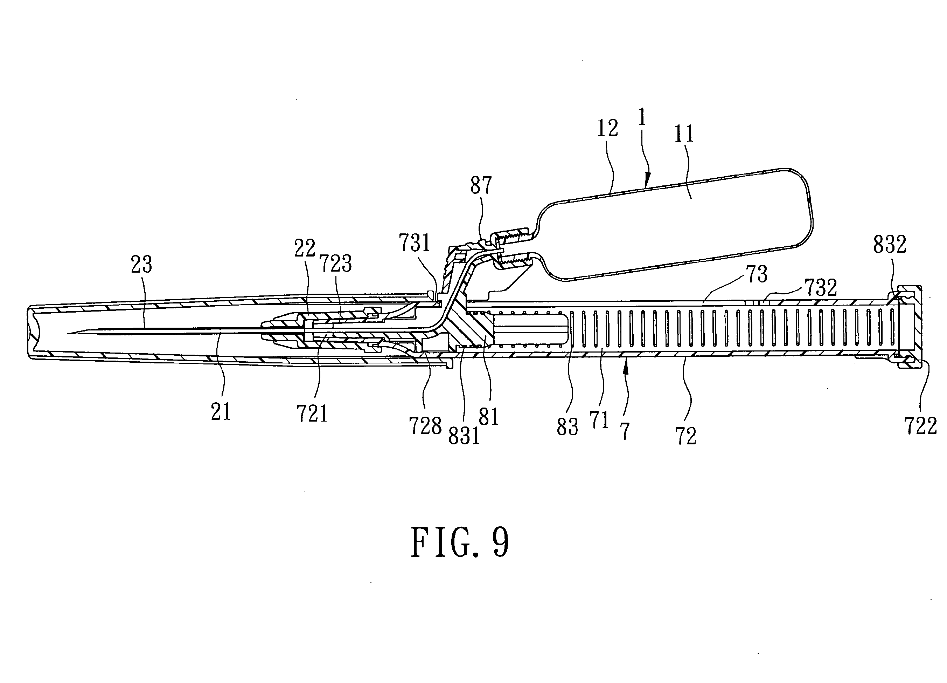

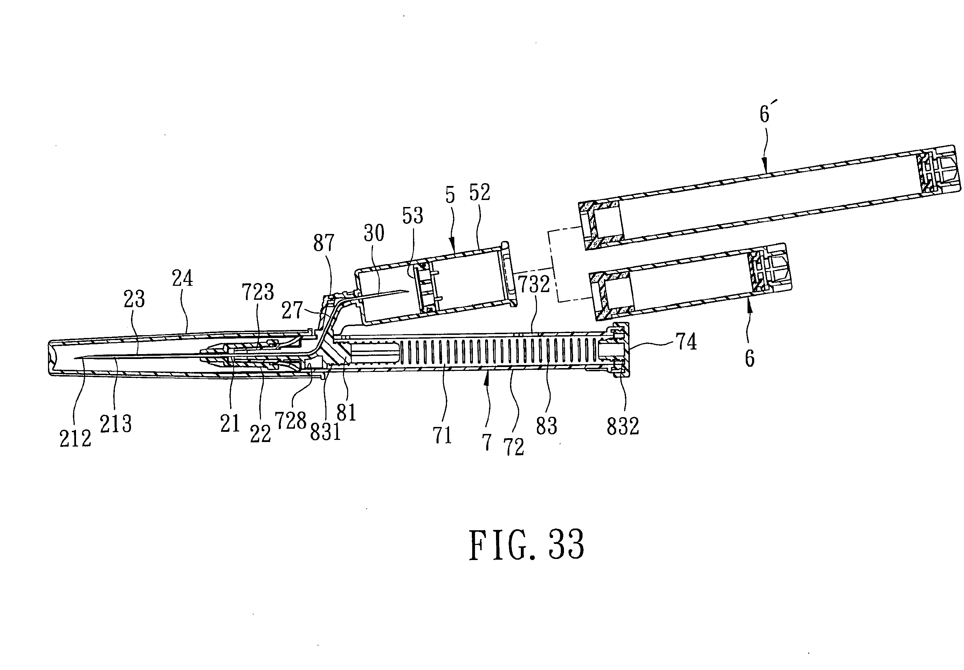

Referring to Fig. 9, the second preferred embodiment of an intravenous catheter introducing device according to this invention is shown to be similar to the first preferred embodiment in construction. In the second embodiment, a biasing spring 83 is received in the guiding groove 71, and has a front spring end 831 which is secured to the force effecting end 81, and a rear spring end 832 which is secured to the tail end portion 722. Alternatively, the rear spring end 832 can be secured to the end cap 74, as shown in Fig. 33. Thus, the biasing spring 83 is tensioned by the needle retracting member 8 when the needle retracting member 8 is in the position of use. In addition, an inner surface of the barrel wall 72 is formed with an annular rib 728 which can reinforce the retention of the needle retracting member 8 in the position of use.

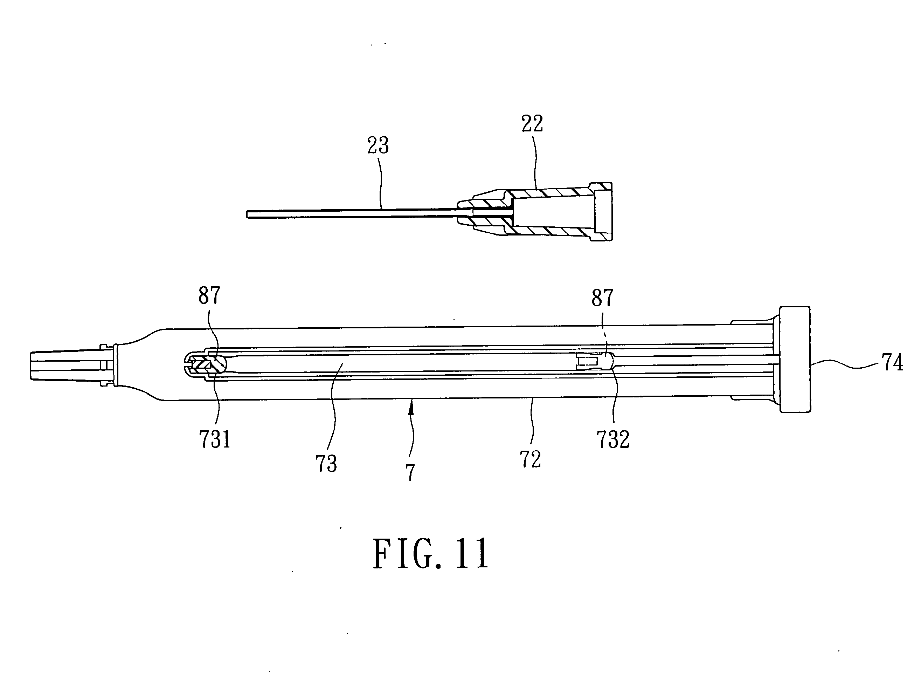

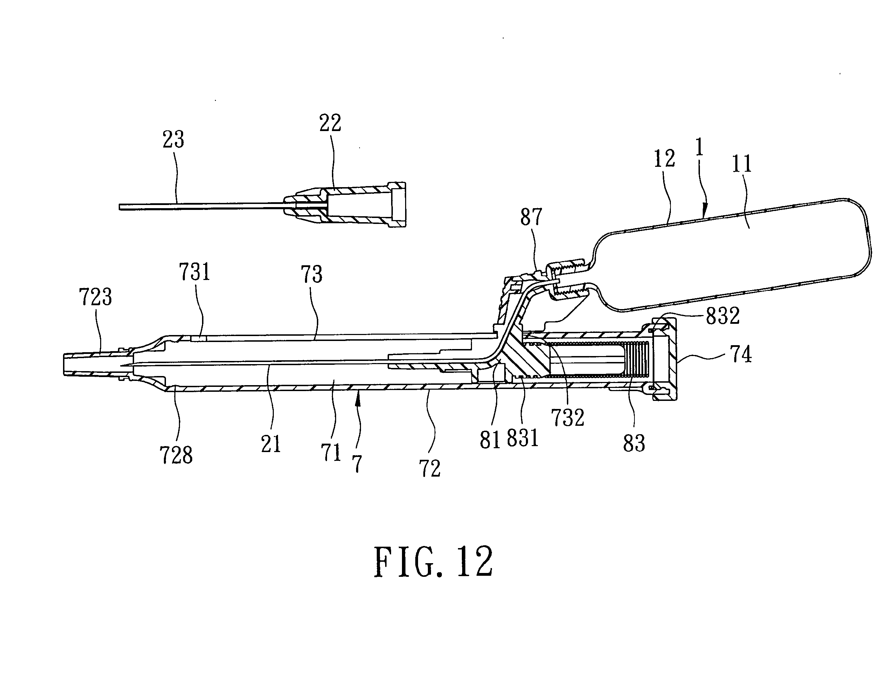

Therefore, referring to Figs. 11 and 12, when the user actuates the shifter 87 with the hand that grips the barrel wall 72 to disengage from the front retaining region 731 and to bring the force effecting end 81 to disengage from the annular rib 728, the biasing spring 83 can bias the needle retracting member 8 to the disposal position for withdrawing the needle cannula 21 into the guiding groove 71.

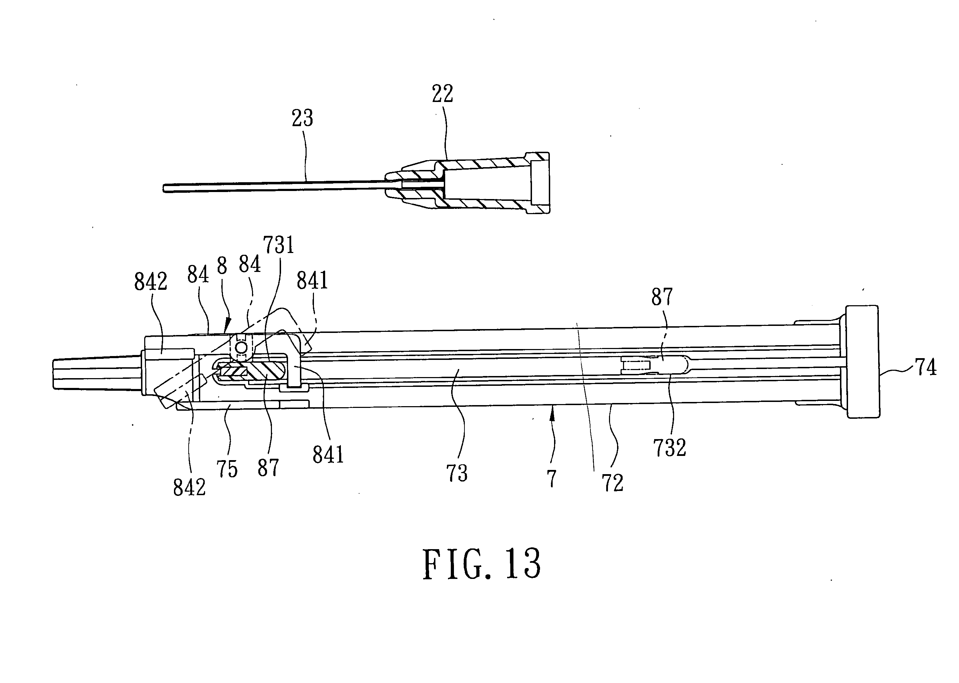

Referring to Fig. 13, the third preferred embodiment of an intravenous catheter introducing device according to this invention is shown to be similar to the second preferred embodiment in construction. In the third embodiment, a gripped member 75 is attached to the barrel wall 72 in the vicinity of the front retaining region 731, and is configured to be held by a user's thumb for moving the shifter 87. A triggering member 84 is pivotally mounted on the barrel wall 72 at a fulcrum point, and has a weight end 841 which is disposed to releasably hold the shifter 87 when the needle retracting member 8 is in the position of use, and a power end 842 which is disposed at an opposite side of the weight end 841 relative to the fulcrum point so as to be actuated to permit the weight end 841 to disengage from the shifter 87, thereby enabling the needle retracting member 8 to move to the disposal position. Thus, after the tubular catheter 23 is introduced into the patient's vein, the user can hold the gripped member 75 with the thumb of the hand that grips the barrel 7, and press the power end 842 with his/her index finger to enable the weight end 841 to disengage from the shifter 87 so as to release the force effecting end 81 that is retained on the front retaining region 731. At this time, the needle retracting member 8 can be moved to the disposal position by means of the biasing force of the biasing spring for withdrawing the needle cannula 21 into the guiding groove 71.

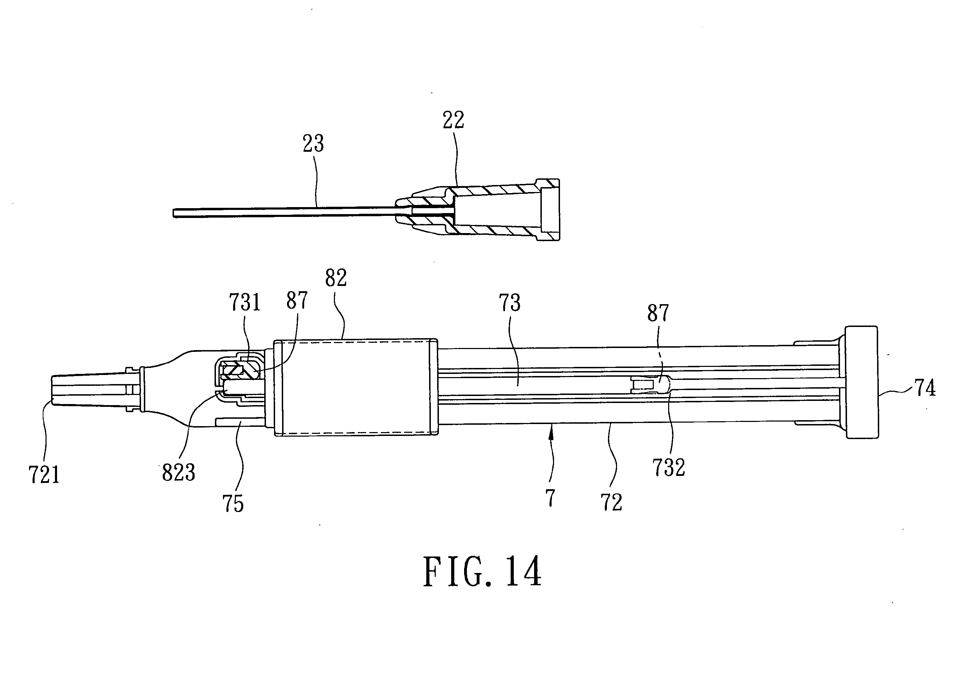

Referring to Fig. 14, the fourth preferred embodiment of an intravenous catheter introducing device according to this invention is shown to be similar to the second preferred embodiment in construction. In the fourth embodiment, the front retaining region 731 extends in a circumferential direction that surrounds the axis (X). In addition, the bracing member 82 has a barrier rib 823 which can be inserted into the front retaining region 731 to guard the shifter 87 against an undesired disengagement from the front retaining region 731. In use, the bracing member 82 is detached from the barrel 7 first, and the catheter introducing operation is subsequently conducted.

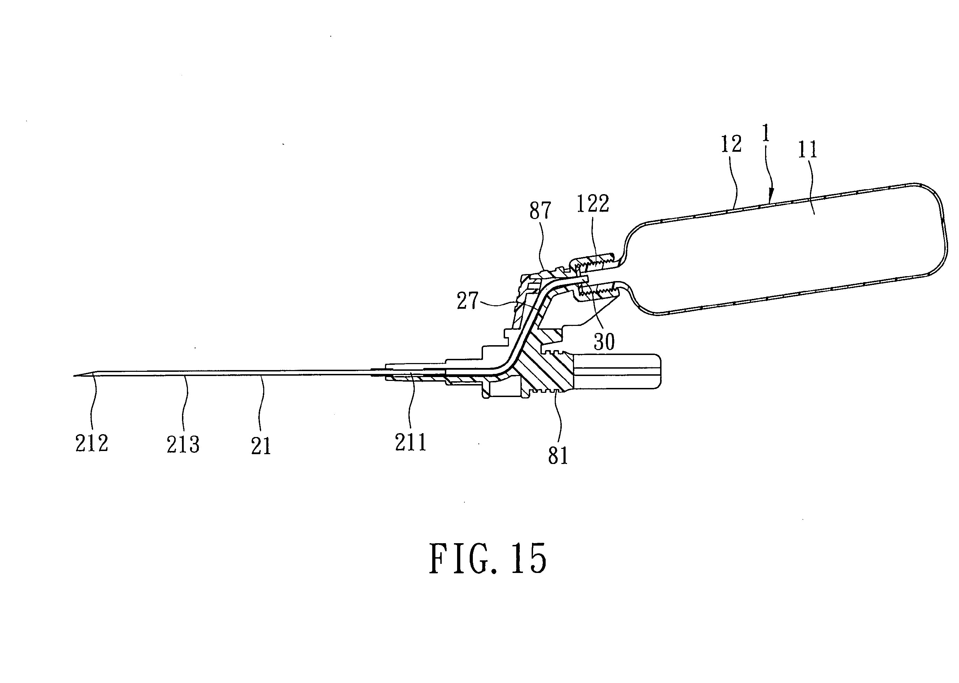

Referring to Fig. 15, the fifth preferred embodiment of an intravenous catheter introducing device according to this invention is shown to be similar to the aforesaid embodiments in construction. In the fifth embodiment, the tubular bent member 27 is in the form of a flexible tube which is light transmissible to permit viewing of blood flowing therethrough.

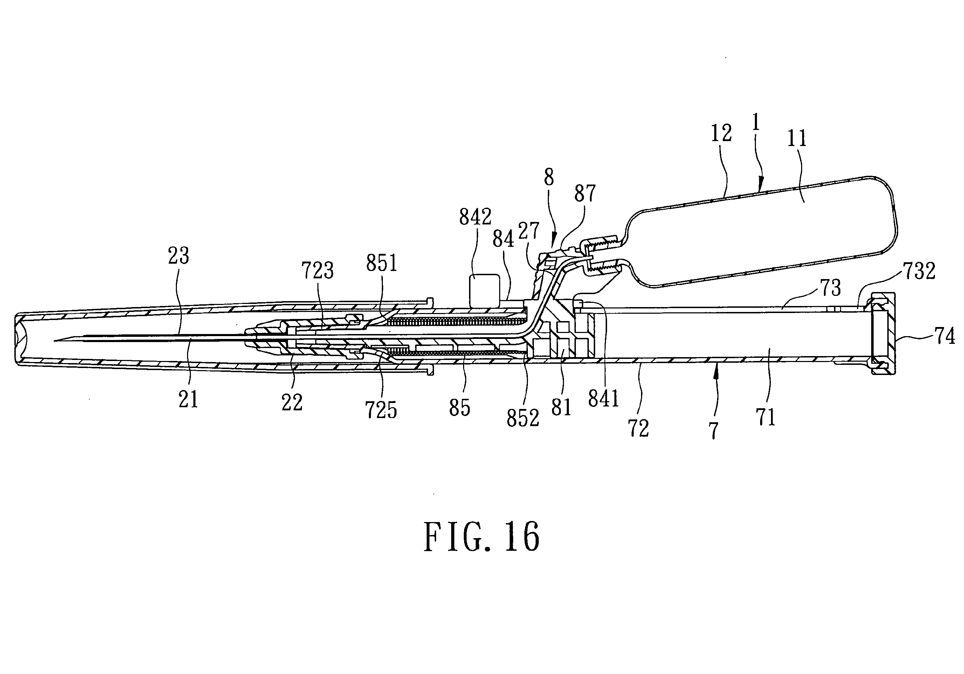

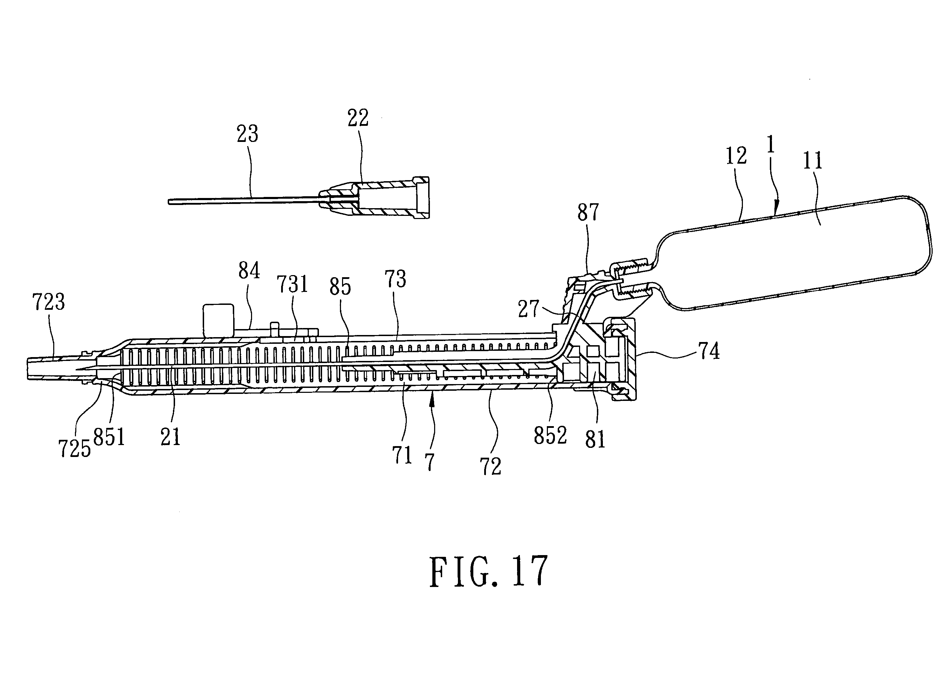

Referring to Figs. 16 and 17, the sixth preferred embodiment of an intravenous catheter introducing device according to this invention is shown to be similar to the third embodiment in construction. In the sixth embodiment, the barrel 5 has a shoulder portion 725 which is disposed proximate to the nose tubular end portion 723 and which faces the guiding groove 71. A biasing spring 85 has front and rear spring ends 851,852 which abut against the shoulder portion 725 and the force effecting end 81, respectively, such that the biasing spring 85 is compressed by the needle retracting member 8 when the needle retracting member 8 is in the position of use.

After completing the introduction of the tubular catheter 23, the user can move the shifter 87 with the hand that grips the barrel wall 72 to permit disengagement of the needle retracting member 8 from the barrel wall 72, whereby the biasing spring 85 can bias the needle retracting member 8 to the disposal position for withdrawing the needle cannula 21 into the guiding groove 71.

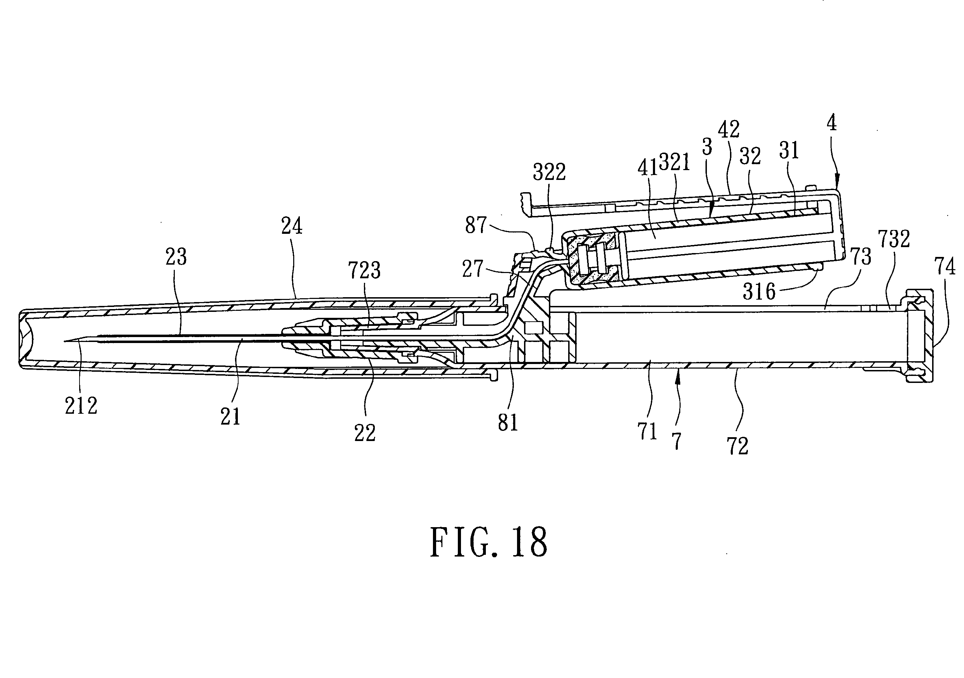

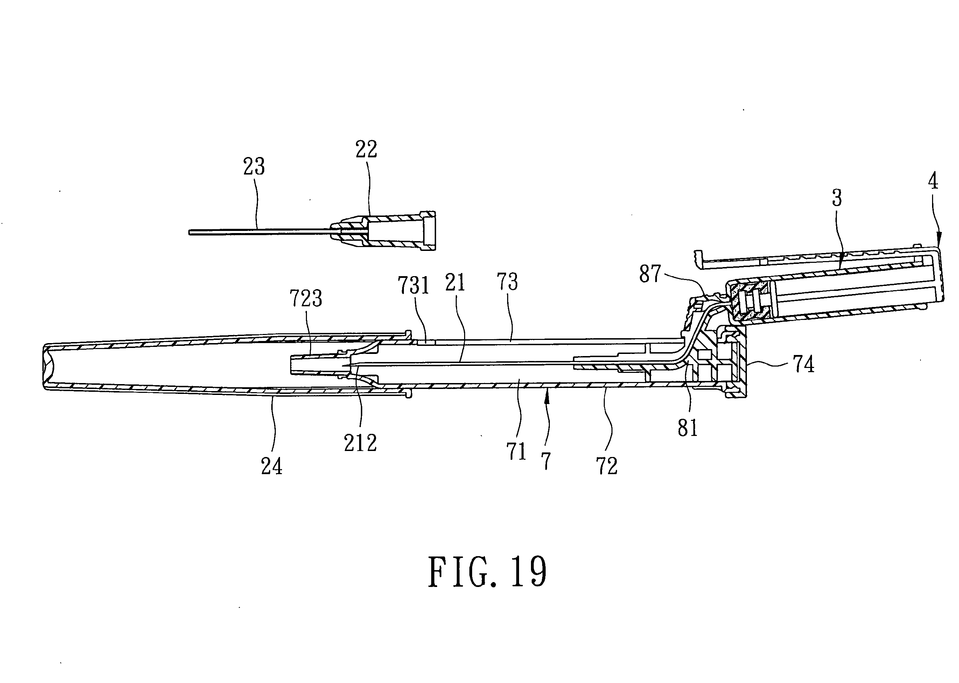

Referring to Figs. 18 and 19, the seventh preferred embodiment of an intravenous catheter introducing device according to this invention is shown to be similar to the first embodiment in construction. In the seventh embodiment, the rear end 316 of the flashback member 3 is formed with a tubular port. A pressure counteracting member 4 includes a plunger 41 and an actuator 42. The plunger 41 is in fluidly tight contact with, and is slidable relative to, an inner surface of the rear wall portion 321 of the surrounding wall 32 so as to generate the reduced pressure in the flashback chamber 31. The actuator 42 is integrally formed with and extends from the plunger 41 to be actuated to move the plunger 41 and to be retained on the rear wall portion 321 in the ready position without the manual force once the tip end 231 has pierced into the skin in search of the vein. In addition, the front wall portion 322 of the surrounding wall 32 is integrally formed with the shifter 87.

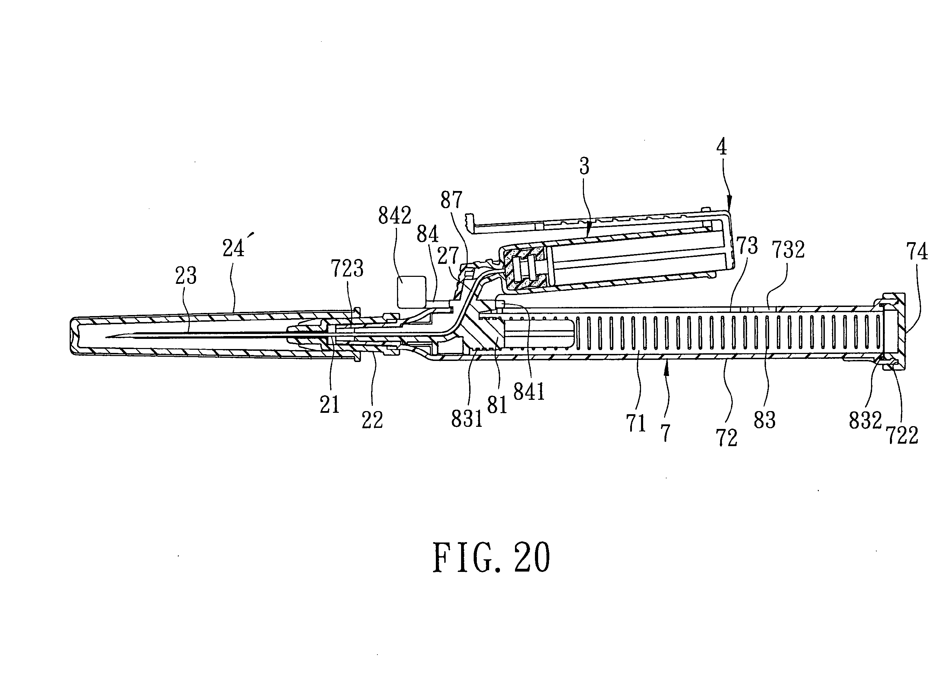

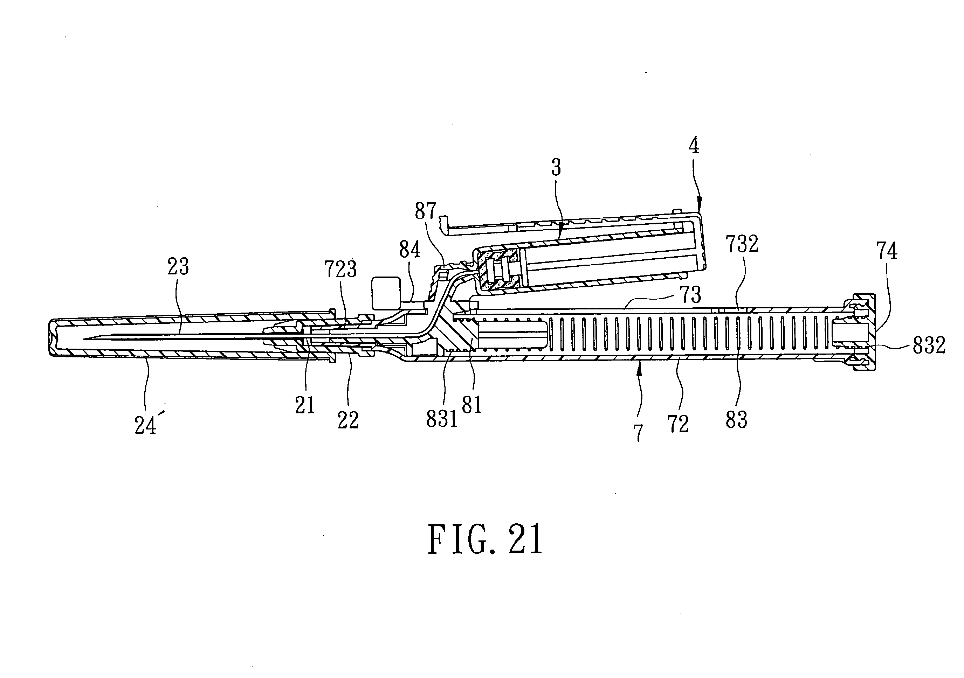

Referring to Fig. 20, the eighth preferred embodiment of an intravenous catheter introducing device according to this invention is shown to be similar to the seventh embodiment in construction. In the eighth embodiment, a biasing spring 83 is received in the guiding groove 71 in the barrel 7, and has a front spring end 831 which is secured to the force effecting end 81 of the needle retracting member 8, and a rear spring end 832 which is secured to the tail end portion 722 of the barrel wall 72. The biasing spring 83 provides the same function as the biasing spring 83 of the aforesaid second embodiment. Alternatively, the rear spring end 832 can be secured to the end cap 74, as shown in Fig. 21. In addition, a triggering member 84 is pivotally mounted on the barrel wall 72 at a fulcrum point, and has weight and power ends 841, 842 opposite to each other. The triggering member 84 provides the same function as the triggering member 84 of the aforesaid third embodiment. Moreover, a tip protector 24' is configured to be detachably sleeved on the catheter hub 22 for shielding the needle cannula 21, the catheter hub 22 and the tubular catheter 23.

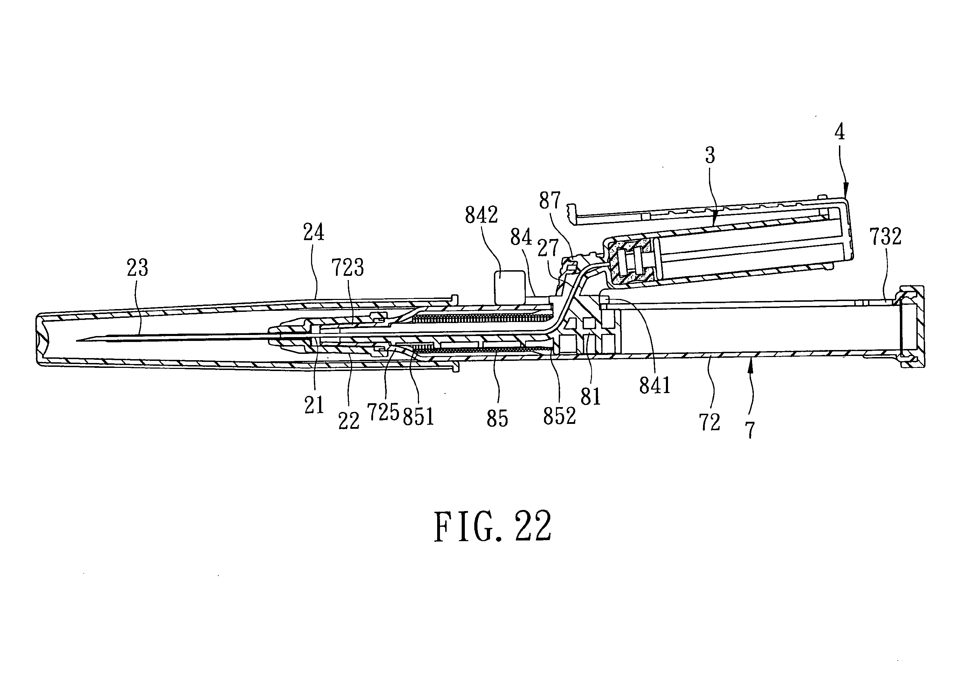

Referring to Fig. 22, the ninth preferred embodiment of an intravenous catheter introducing device according to this invention is shown to be similar to the seventh embodiment in construction. In this embodiment, a biasing spring 85 has front and rear spring ends 851,852 which abut against the shoulder portion 725 of the barrel 72 and the force effecting end 81, respectively, such that the biasing spring 85 is compressed by the needle retracting member 8 when the needle retracting member 8 is in the position of use. The biasing spring 85 provides the same function as the biasing spring of the aforesaid sixth embodiment. In addition, a triggering member 84 is pivotally mounted on the barrel wall 72 at a fulcrum point, and has weight and power ends 841, 842 opposite to each other. The triggering member 84 provides the same function as the triggering member of the aforesaid third embodiment.

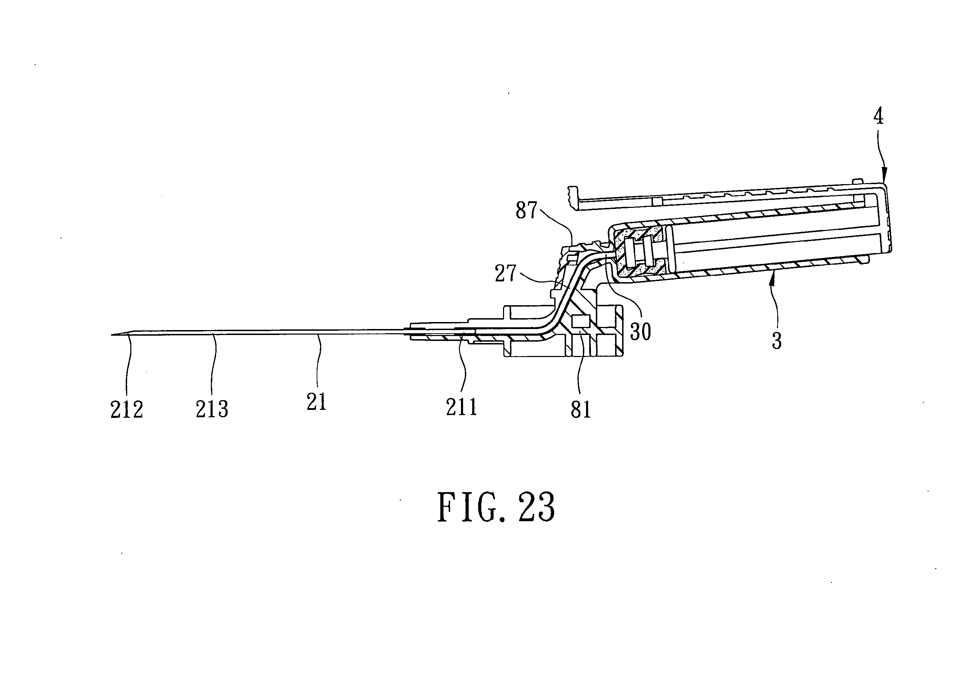

Referring to Fig. 23, the tenth preferred embodiment of an intravenous catheter introducing device according to this invention is shown to be similar to the aforesaid seventh to ninth embodiments in construction. In the tenth embodiment, the tubular bent member 27 is in the form of a flexible tube which is light transmissible to permit viewing of blood flowing therethrough.

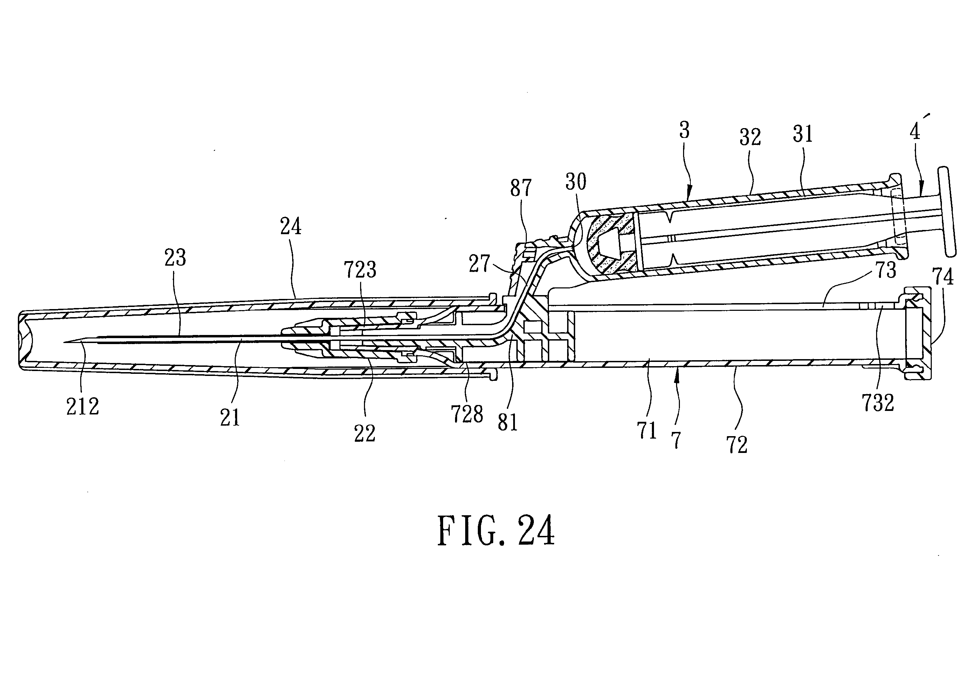

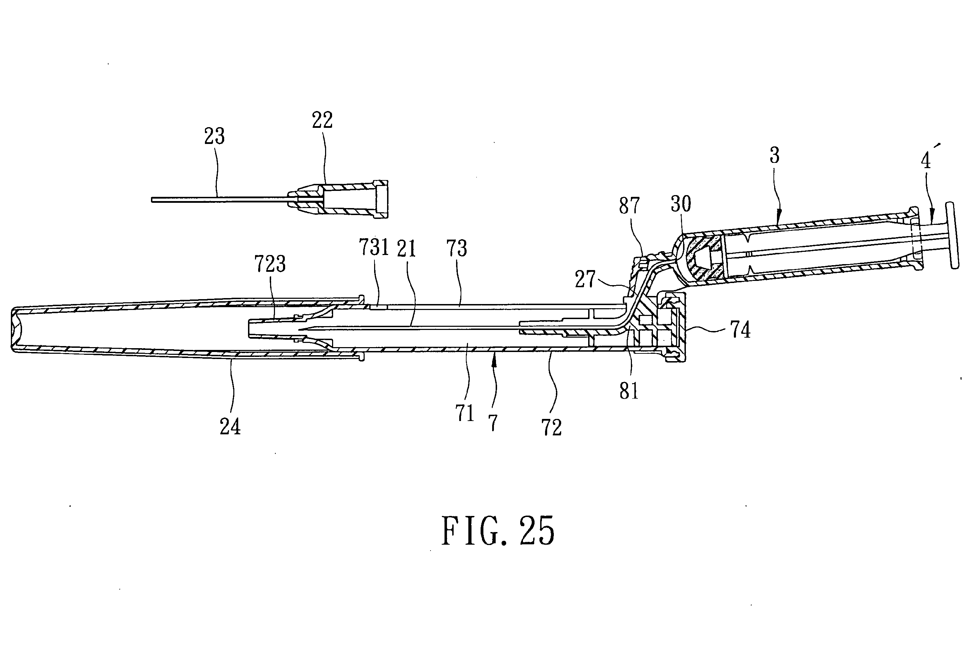

Referring to Figs. 24 and 25, the eleventh preferred embodiment of an intravenous catheter introducing device according to this invention is shown to be similar to the aforesaid seventh embodiment in construction. In the eleventh embodiment, instead of the plunger 41 and the actuator 42, a plunger 4' which is configured to be the same as that of a known syringe is in fluidly tight contact with, and is slidable relative to, an inner surface of the surrounding wall 32 of the flashback member 3 so as to generate the reduced pressure in the flashback chamber 31. The user can pull the plunger 4' rearwardly to generate the reduced pressure so as to draw a little blood into the flashback chamber 11 for facilitating the catheter introducing operation.

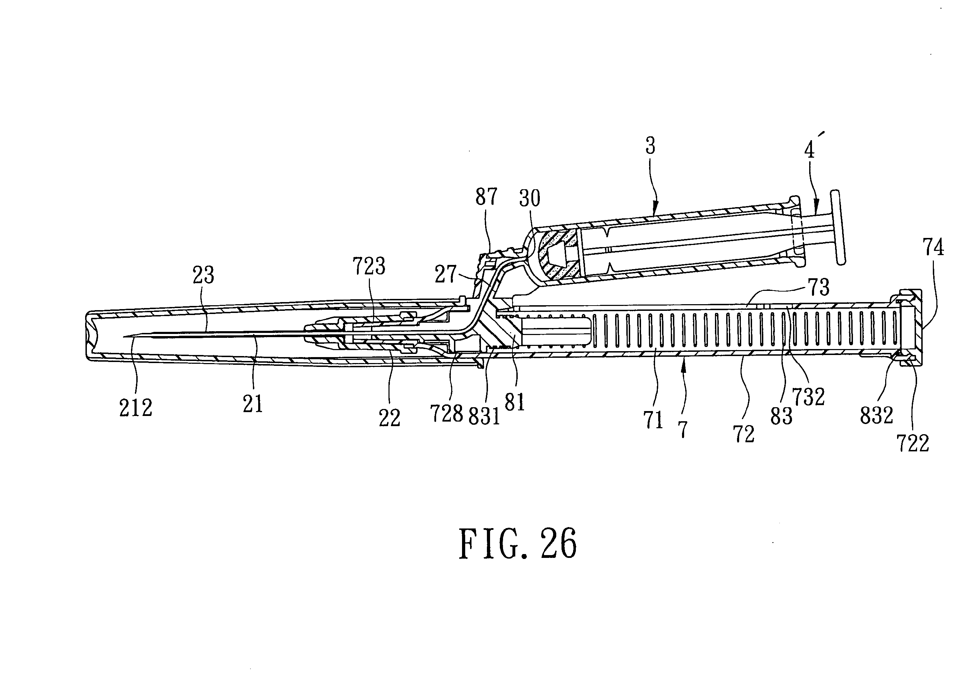

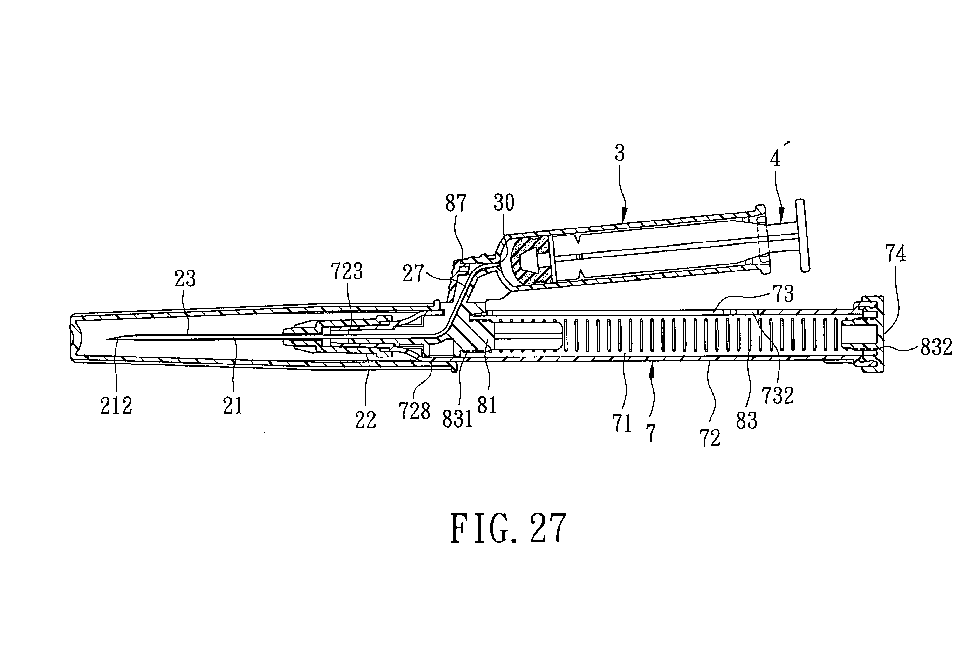

Referring to Figs. 26 and 27, the twelfth preferred embodiment of an intravenous catheter introducing device according to this invention is shown to be similar to the eleventh embodiment in construction. In the twelfth embodiment, a biasing spring 83 is received in the guiding groove 71 in the barrel 7, and has a front spring end 831 which is secured to the force effecting end 81 of the needle retracting member 8, and a rear spring end 832 which is secured to the tail end portion 722 of the barrel wall 72. The biasing spring 83 provides the same function as the biasing spring of the aforesaid second embodiment. Alternatively, the rear spring end 832 can be secured to the end cap 74, as shown in Fig. 27.

ReferringtoFig. 28, the thirteenth preferred embodiment of an intravenous catheter introducing device according to this invention is shown to be similar to the eleventh embodiment in construction. In this embodiment, a biasing spring 85 has front and rear spring ends 851, 852 which abut against the shoulder portion 725 of the barrel 72 and the force effecting end 81, respectively, such that the biasing spring 85 is compressed by the needle retracting member 8 when the needle retracting member 8 is in the position of use. The biasing spring 85 provides the same function as the biasing spring of the aforesaid sixth embodiment.

ReferringtoFig. 29, the fourteenth preferred embodiment of an intravenous catheter introducing device according to this invention is shown to be similar to the aforesaid eleventh to thirteenth embodiments in construction. In the fourteenth embodiment, the tubular bent member 27 is in the form of a flexible tube which is light transmissible to permit viewing of blood flowing therethrough.

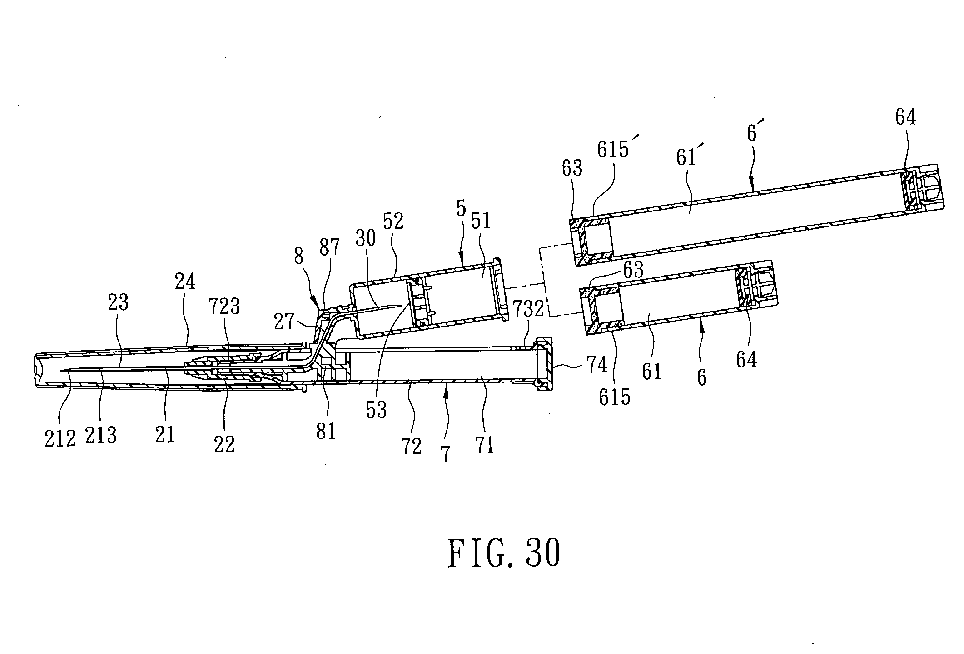

Referring to Fig. 30, the fifteenth preferred embodiment of an intravenous catheter introducing device according to this invention is shown to be similar to the aforesaid seventh embodiment in construction. In the fifteenth embodiment, a shell member 5 is connected to and is disposed rearwardly of the shifter 87 to surround the tubular outlet member 30, and defines a receiving chamber 51 to detachable receive the flashback member 6,6'. An air-permeable and liquid-impermeable partition member 53 is disposed in the receiving chamber 51 and is pierceable by the tubular outlet member 30. The partition member 53 is configured to air-tightly engage and to be movable relative to a shell wall of the shell member 5. Thus, once the partition member 53 is pushed by the flashback member 6,6' to permit the tubular outlet member 30 to pierce through the partition member 53, a subsequent pushing action of the flashback member 6,6' results in passing of the tubular outlet member 30 through the front end 615 , 615' of the flashback member 6,6' so as to fluidly communicate the flashback chamber 61,61' with the needle cannula 21. Front and rear sealing members 63,64 are disposed to seal the front and rear ends of the flashback member 6,6' so as to confine the flashback chamber 61, 61' . Fluid with a reduced pressure is disposed in the flashback chamber 61,61'.

In use, the user can pierce a target vein of a patient with the tip end 212 so that blood flows into the receiving chamber 51 to indicate a successful introduction of the tubular catheter 23. If blood is not observed in the receiving chamber 51, the user can insert the flashback member 6,6' into the receiving chamber 51. Subsequently, the partition member 53 is pushed by the flashback member 6,6' to permit the tubular outlet member 30 to pierce through the partition member 53. A subsequent pushing action of the flashback member 6,6' results in passing of the tubular outlet member 30 through the front sealing member 63 so as to fluidly communicate the flashback chamber 61,61' with the needle cannula 21, thereby facilitating the flow of blood into the flashback chamber 61,61' by means of the reduced pressure. Moreover, the blood can be collected into the flashback chamber 61,61' for taking a blood sample. The flashback member 61' , which has a longer flashback chamber 61, is used for collecting a larger amount of blood sample as required.

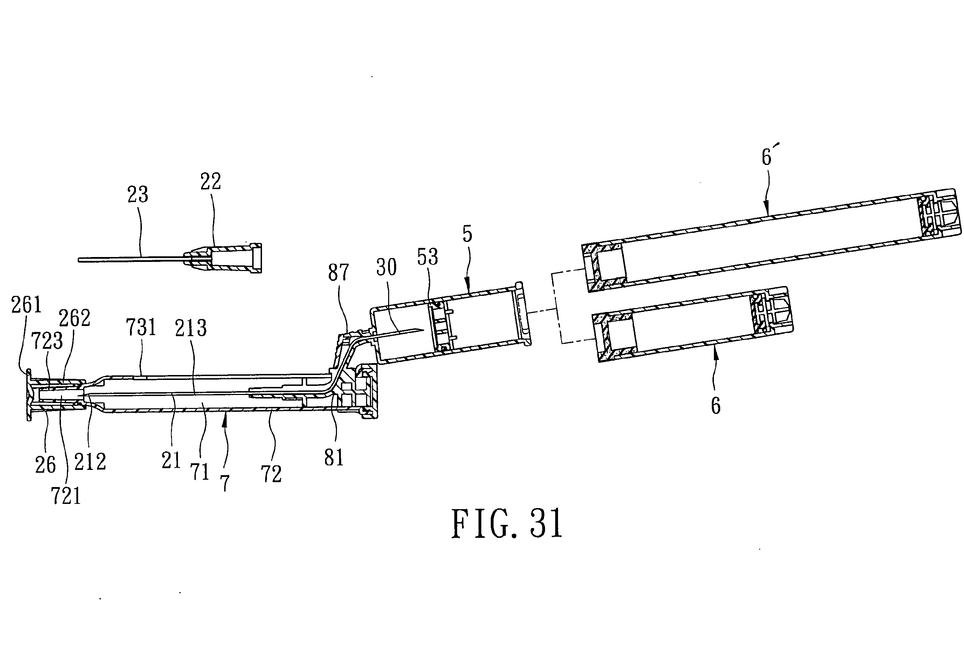

Referring to Fig. 31, after the tip end 212 of the needle cannula 21 is withdrawn into the guiding groove 71, a protecting cap 26 has a cap surrounding wall 262 sleeved on the nose tubular end portion 723, and a cap end wall 261 disposed to close the passage 721.

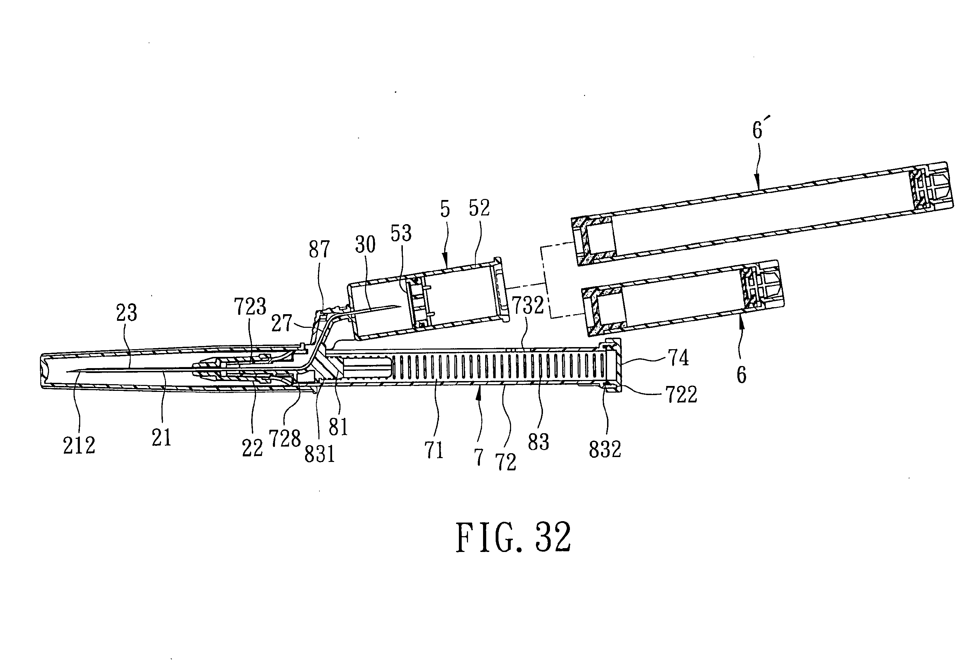

Referring to Figs. 32 and 33, the sixteenth preferred embodiment of an intravenous catheter introducing device according to this invention is shown to be similar to the fifteenth embodiment in construction. In the sixteenth embodiment, a biasing spring 83 is received in the guiding groove 71 in the barrel 7, and has a front spring end 831 which is secured to the force effecting end 81 of the needle retracting member 8, and a rear spring end 832 which is secured to the tail end portion 722 of the barrel wall 72. The biasing spring 83 provides the same function as the biasing spring of the aforesaid second embodiment. Alternatively, the rear spring end 832 can be secured to the end cap 74, as shown in Fig. 33.

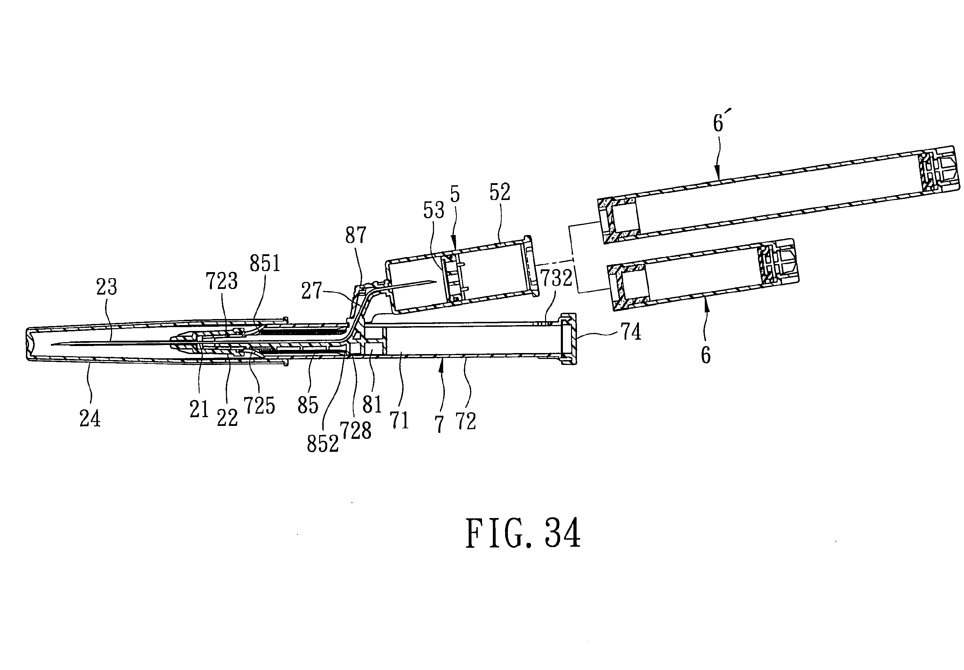

Referring to Fig. 34, the seventeenth preferred embodiment of an intravenous catheter introducing device according to this invention is shown to be similar to the fifteenth embodiment in construction. In this embodiment, a biasing spring 85 has front and rear spring ends 851, 852 which abut against the shoulder portion 725 of the barrel 72 and the force effecting end 81, respectively, such that the biasing spring 85 is compressed by the needle retracting member 8 when the needle retracting member 8 is in the position of use. The biasing spring 85 provides the same function as the biasing spring of the aforesaid sixth embodiment.

ReferringtoFig. 35, the eighteenth preferred embodiment of an intravenous catheter introducing device according to this invention is shown to be similar to the aforesaid fifteenth to seventeenth embodiments in construction. In the eighteenth embodiment, the tubular bent member 27 is in the form of a flexible tube which is light transmissible to permit viewing of blood flowing therethrough.

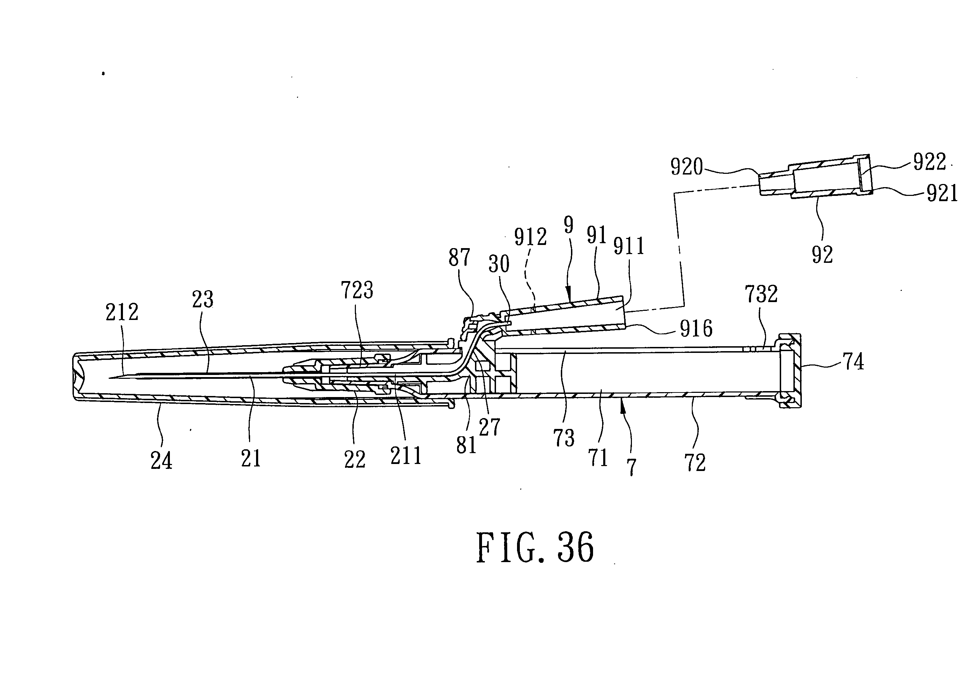

Referring to Fig. 36, the nineteenthpreferredembodiment of an intravenous catheter introducing device according to this invention is shown to be similar to the first embodiment in construction. In the nineteenth embodiment, the rear end 916 of the flashback member 9 is formed with a tubular port 911. A tubular hub 92 has a front hub segment 920 which is detachably inserted into the tubular port 911, a rear port segment 921 opposite to the front hub segment 920 in the longitudinal direction, and an air-permeable member 922 which engages and closes the rear port segment 921. In addition, the flashback member 9 has a grip 912 which is disposed on the front wall portion of the surrounding wall 91 for gripping by a user's finger.

In this embodiment, the flashback member 9 can be connected to either one of the above types of the flashback member 1 of the first embodiment, the flashback members 3 of the seventh and eleventh embodiments, and the flashback members 6,6' of the fifteenth embodiment after the tubular hub 92 is removed from the tubular port 911 so as to provide a blood drawing function to facilitate the catheter introducing operation.

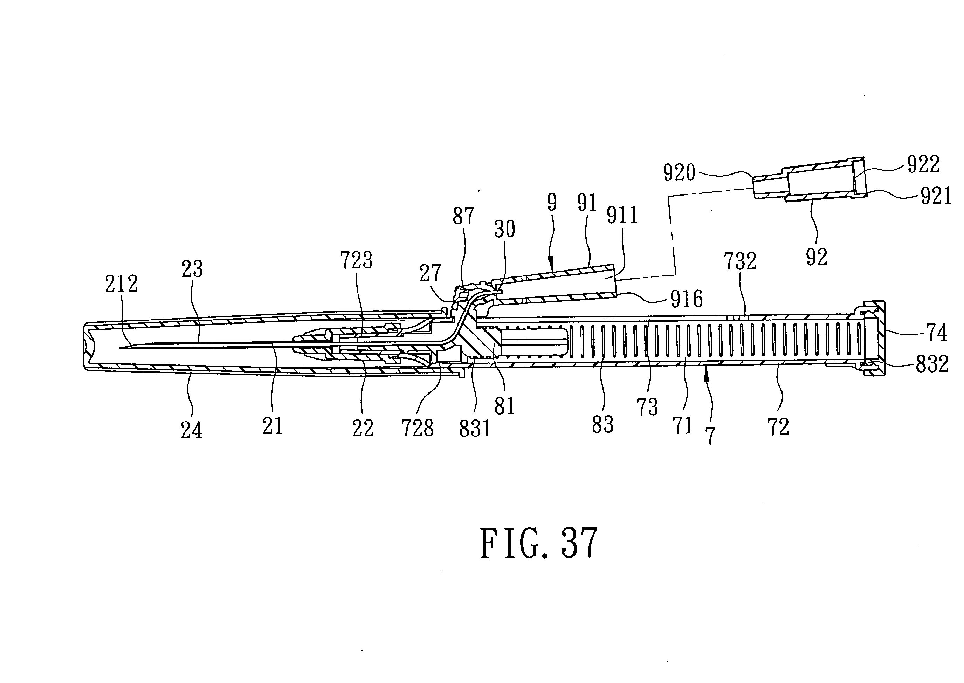

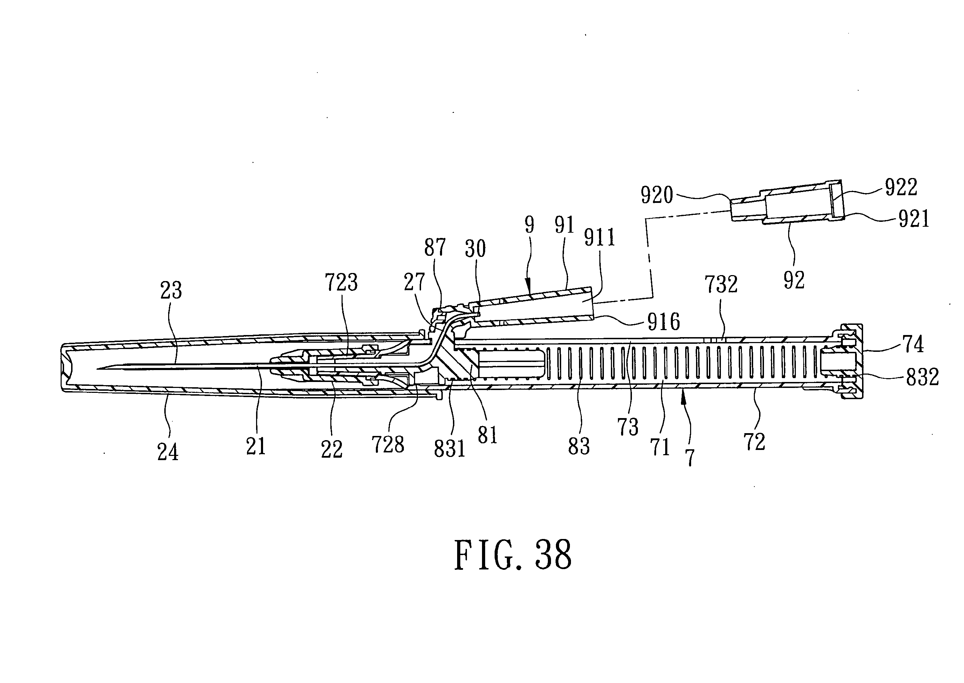

Referring to Figs. 37 and 38, the twentieth preferred embodiment of an intravenous catheter introducing device according to this invention is shown to be similar to the nineteenth embodiment in construction. In the twentieth embodiment, a biasing spring 83 is received in the guiding groove 71 in the barrel 7, and has a front spring end 831 which is secured to the force effecting end 81 of the needle retracting member 8, and a rear spring end 832 which is secured to the tail end portion 722 of the barrel wall 72. The biasing spring 83 provides the same function as the biasing spring of the aforesaid second embodiment. Alternatively, the rear spring end 832 can be secured to the end cap 74, as shown in Fig. 38.

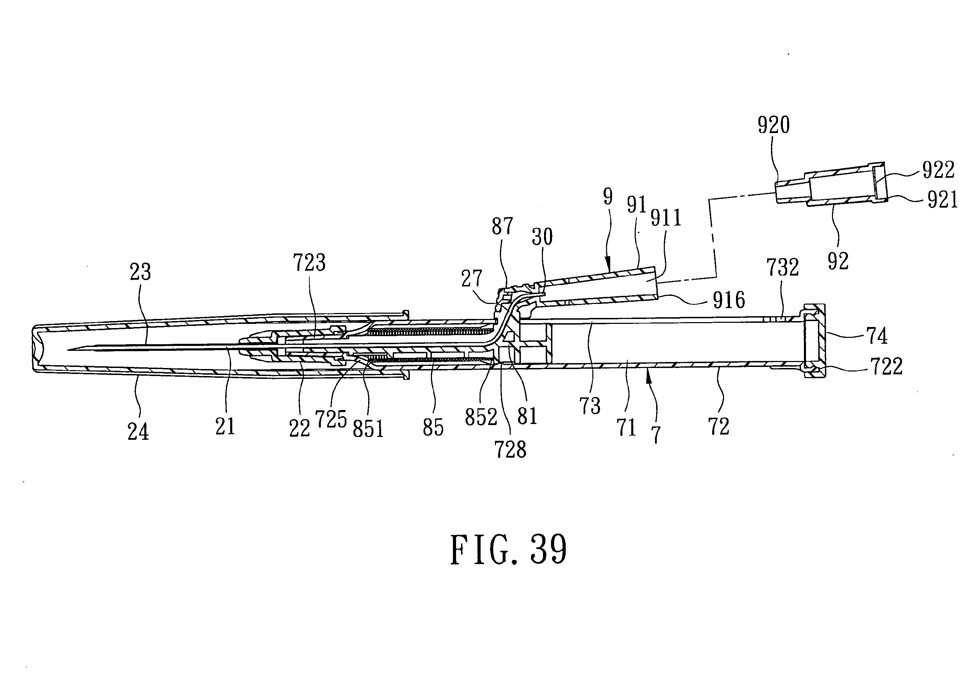

Referring to Fig. 39, the twenty-first preferred embodiment of an intravenous catheter introducing device according to this invention is shown to be similar to the nineteenth embodiment in construction. In this embodiment, a biasing spring 85 has front and rear spring ends 851,852 which abut against the shoulder portion 725 of the barrel 72 and the force effecting end 81, respectively, such that the biasing spring 85 is compressed by the needle retracting member 8 when the needle retracting member 8 is in the position of use. The biasing spring 85 provides the same function as the biasing spring of the aforesaid sixth embodiment.

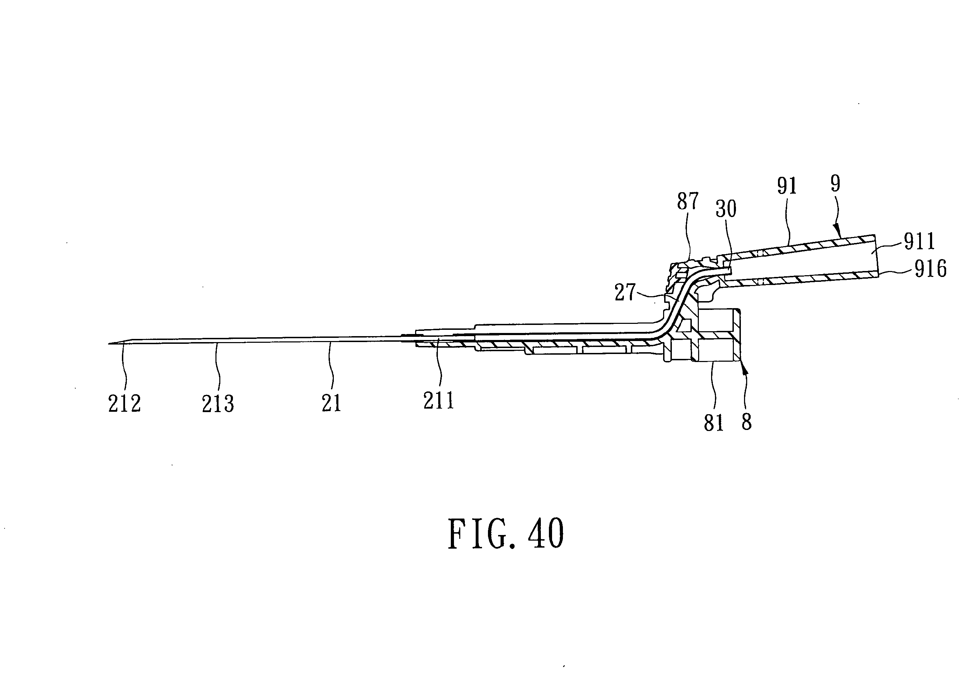

Referring to Fig. 40, the twenty-second preferred embodiment of an intravenous catheter introducing device according to this invention is shown to be similar to the aforesaid nineteenth to twenty-first embodiments in construction. In the eighteenth embodiment, the tubular bent member 27 is in the form of a flexible tube which is light transmissible to permit viewing of blood flowing therethrough.

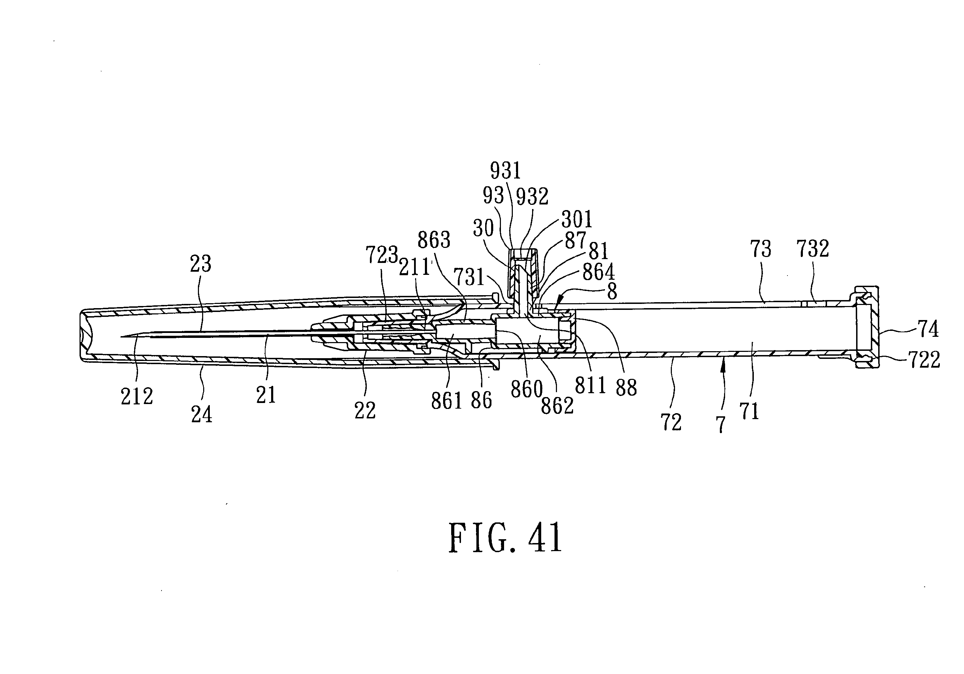

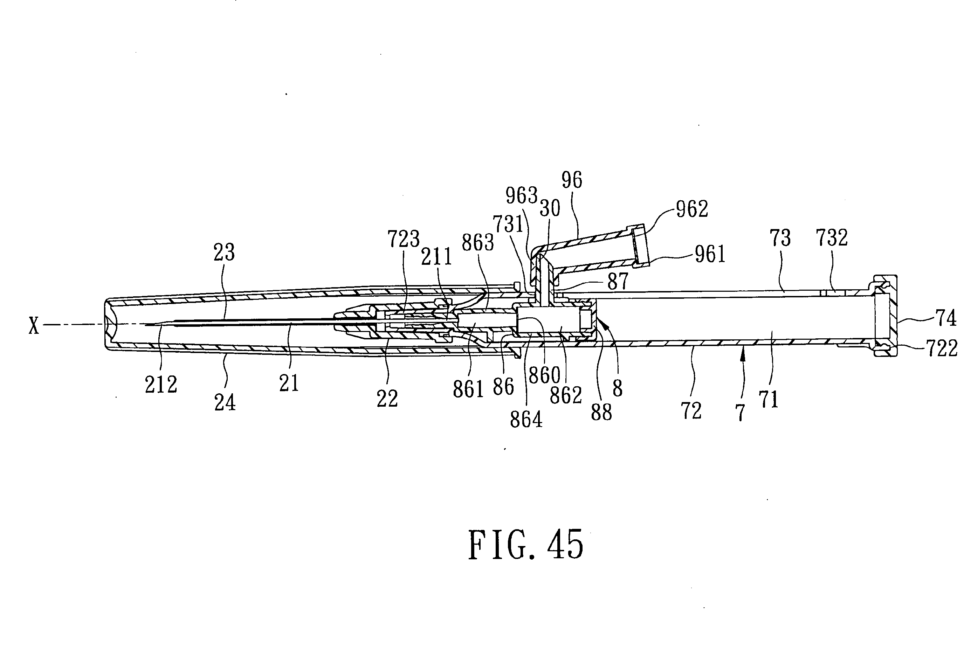

Referring to Figs. 41 to 55, in the following embodiments, an intravenous catheter introducing device according to this invention is shown to be similar to the first embodiment in construction. The difference resides in that the tubular outlet member 30 is integrally formed with the shifter 87 of the needle retracting member 8, the force effecting end 81 has an internal port 811 which is disposed downstream of the rear connecting end 211 of the needle cannula 21, and the shifter 87 is configured to be a hollow stem so as to communicate the tubular outlet member 30 with the internal port 811. The tubular outlet member 30 has an upper region and a lower region which is configured to be shorter than the upper region so as to form the inclined end wall 301.

In addition, the flashback member 86 is disposed in the barrel wall 72, and has a surrounding wall including a rear wall portion 864 and a front wall portion 863. The rear wall portion 864 is integrally formed with the needle retracting member 8, and is disposed in the guiding groove 71 in the barrel wall 72 to permit the hollow stem of the shifter 87 to extend radially and outwardly of the barrel wall 72 through the elongated guideway 73. The rear wall portion 864 defines therein a passageway 862 which is disposed upstream of the internal port 811. The front wall portion 863 is coupled with the rear connecting end 211, and defines a flashback chamber 861 such that the flashback chamber 861 is disposed downstream of the needle cannula 21. Moreover, the front wall portion 863 can be made from a light-transmissible material and formed as a convex so as to enable the user to clearly observe the blood flowing therethrough. A flow-barrier member 860 made from a liquid-impermeable and air-permeable material is disposed to partition the flashback chamber 861 and the passageway 862. A cover cap 88 is disposed to cover a rear opened end of the rear wall portion 864.

In the twenty-third preferred embodiment as shown in Fig. 41, a first tubular member 93 includes a proximate tubular end which is detachably sleeved on and which surrounds the tubular outlet member 30, an extended portion which extends from the proximate tubular end in a direction substantially parallel to the tubular outlet member 30 to terminate at a distal tubular end 931, and an air-permeable member 932 which is disposed to engage the extended portion such that the blood sample drained out of the tubular outlet member 30 is prevented from reaching the distal tubular end 931.

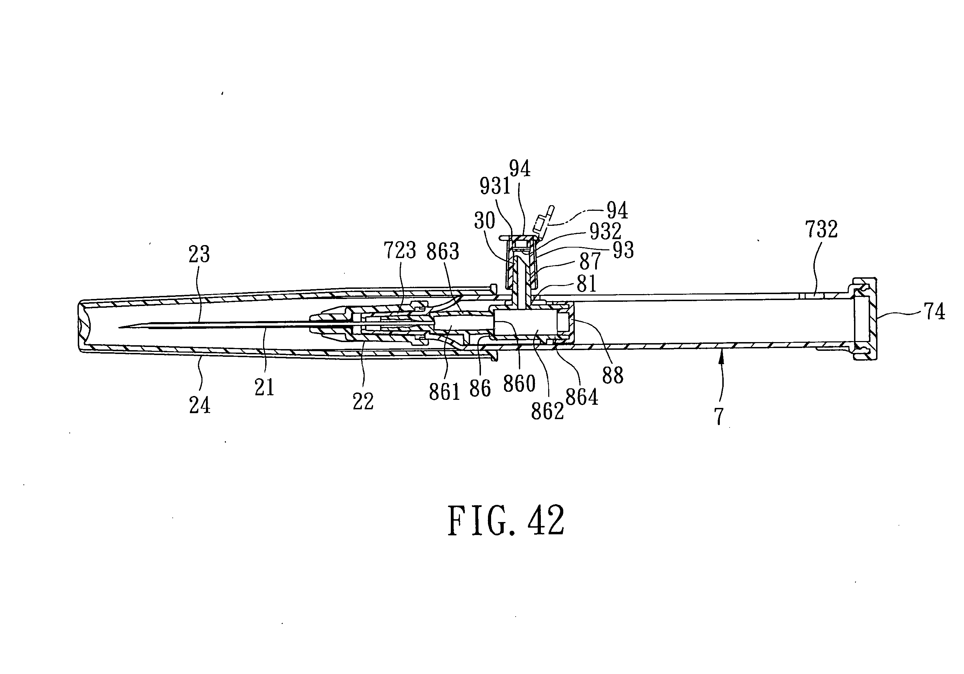

Alternatively, as shown in Fig. 42, an end cap 94 may be further included, and is hinged to the distal tubular end 931 of the first tubular member 93 to openably close the distal tubular end 931.

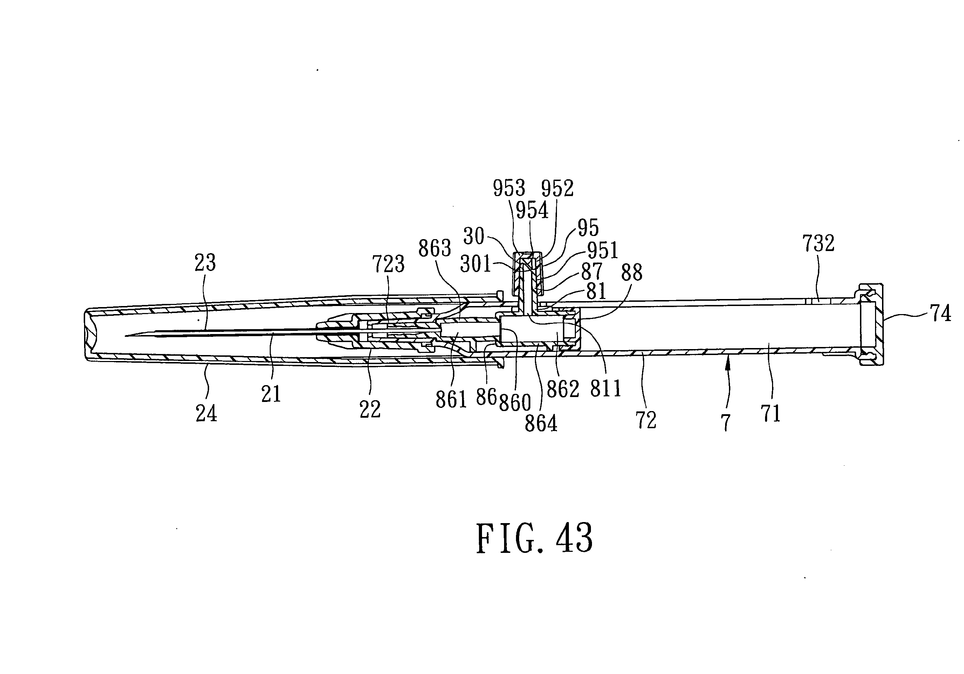

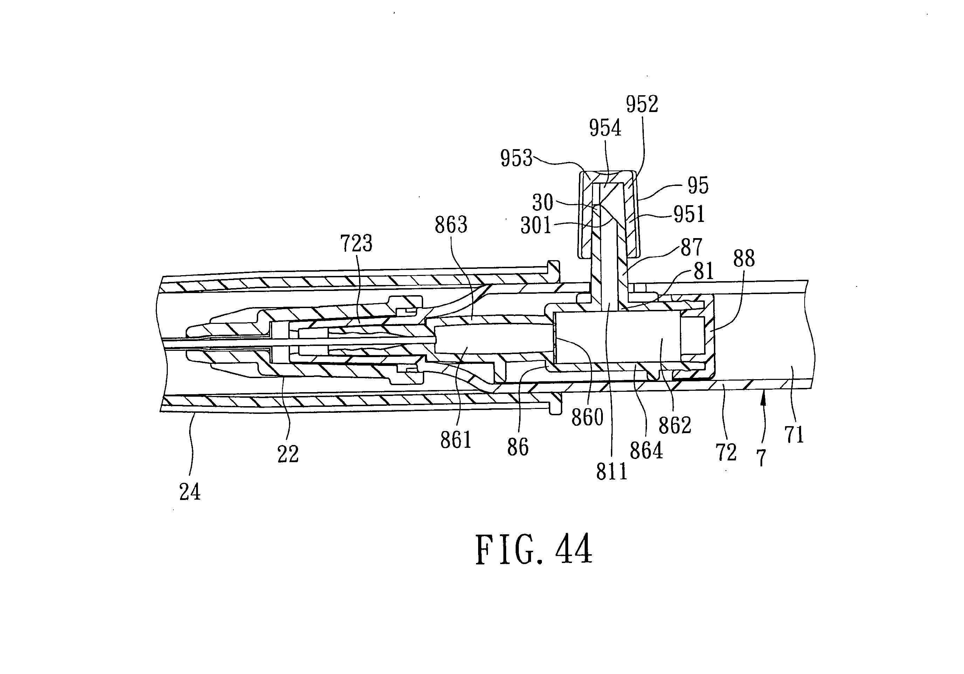

As shown in Figs. 43 and 44, the twenty-fourth preferred embodiment of an intravenous catheter introducing device according to this invention is shown to be similar to the twenty-third embodiment in construction. Instead of the first tubular member 93, an end cover 95 is provided, which includes a tubular wall 951 that is detachably sleeved on and that surrounds the tubular outlet member 30, and that extends in a direction parallel to the tubular outlet member 30 to terminate at a tubular edge 952, and an end wall 953 that is connected to and integrally formed with the tubular edge 952 such that the end wall 953 confronts the internal port 811. A sloping abutment 954 extends from the end wall 953, and is configured to mate with the inclined end wall 301 of the outlet tubular member 30 so as to establish a fluid-tight engagement between the tubular outlet member 30 and the end cover 95. Thus, when the end cover 95 is rotated to permit a lowest portion of the sloping abutment 954 to abut against the upper region of the tubular outlet member 30, the fluid-tight engagement can be released so as to permit escape of air from the flashback chamber 861 through the tubular outlet member 30 during the introduction of the tubular catheter 21.

AsshowninFig. 45, thetwenty-fifthpreferredembodiment of an intravenous catheter introducing device according to this invention is shown to be similar to the twenty-third embodiment in construction. Instead of the first tubular member 93, a second tubular member 96 is provided, which includes a proximate tubular end 963 that is detachably sleeved on and that surrounds the tubular outlet member 30, an extended portion that extends from and that is bent relative to the proximate tubular end 963 in a direction substantially parallel to the axis (X) and that terminates at a distal tubular end 961, and an air-permeable member 962 engaging and closing the distal tubular end 961.

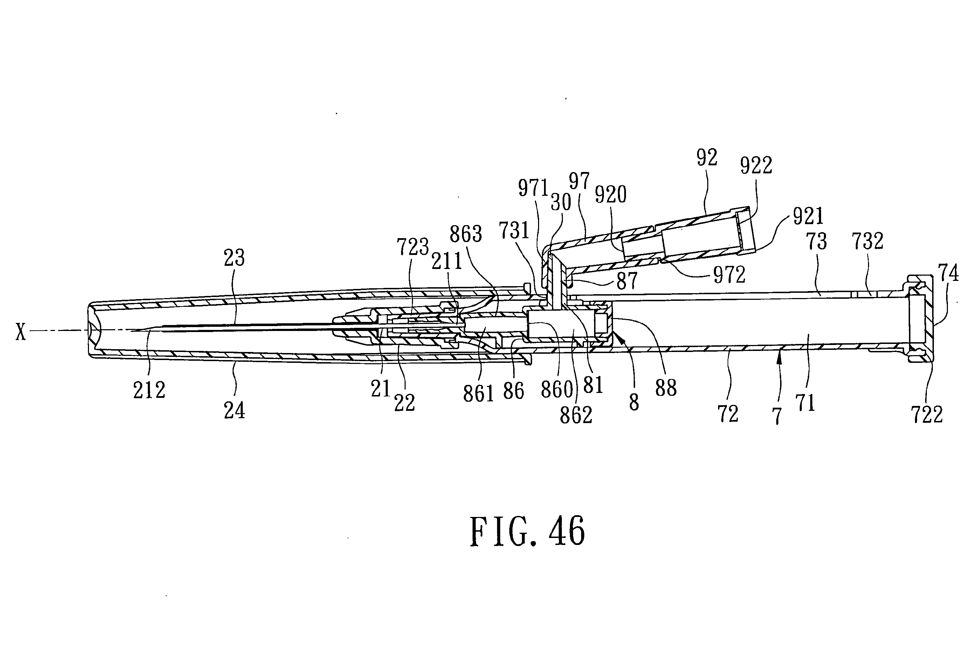

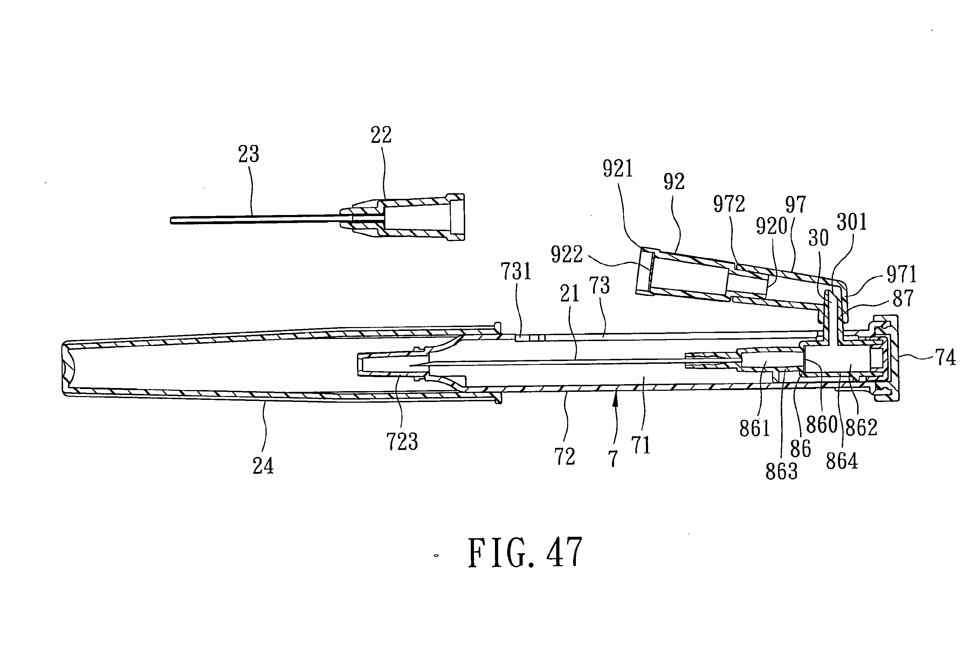

As shown in Figs. 46 and 47, the twenty-sixth preferred embodiment of an intravenous catheter introducing device according to this invention is shown to be similar to the twenty-third embodiment in construction. Instead of the first tubular member 93, a third tubular member 97 is provided, which includes a proximate tubular end 971 that is detachably sleeved on and that surrounds the tubular outlet member 30, and an extended portion that extends from and that is bent relative to the proximate tubular end 971 in a direction substantially parallel to the axis (X) and that terminates at a distal tubular end 972. In addition, a tubular hub 92 has a front connecting end 920 which is detachably inserted into the distal tubular end 972, a rear connecting port 921 opposite to the front connecting end 920 in the longitudinal direction, and an air-permeable member 922 disposed to engage and close the rear connecting port 921.

In use, the tubular hub 92 is removed, and the flashback member 9 can be connected to either one of the above types of the flashback member 1 of the first embodiment, the flashback members 3 of the seventh and eleventh embodiments, and the flashback members 6,6' of the fifteenth embodiment so as to provide a blood drawing function to facilitate the catheter introducing operation. Moreover, the third tubular member 97 can also be removed such that the tubular outlet member 30 is connected to a suitable flashback member 1,3,6,6' with a bent front portion.

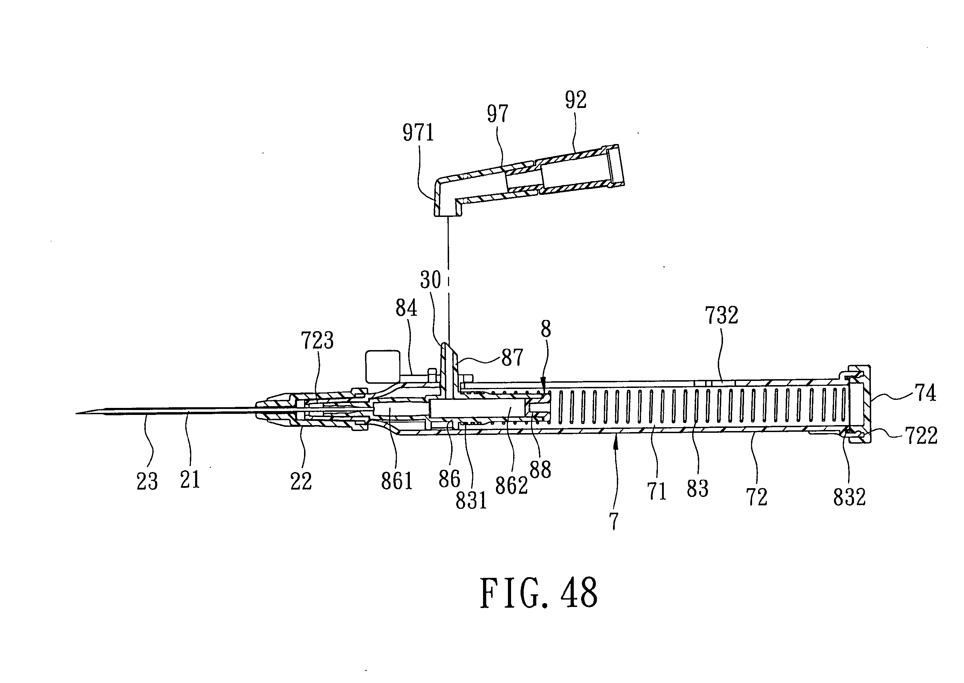

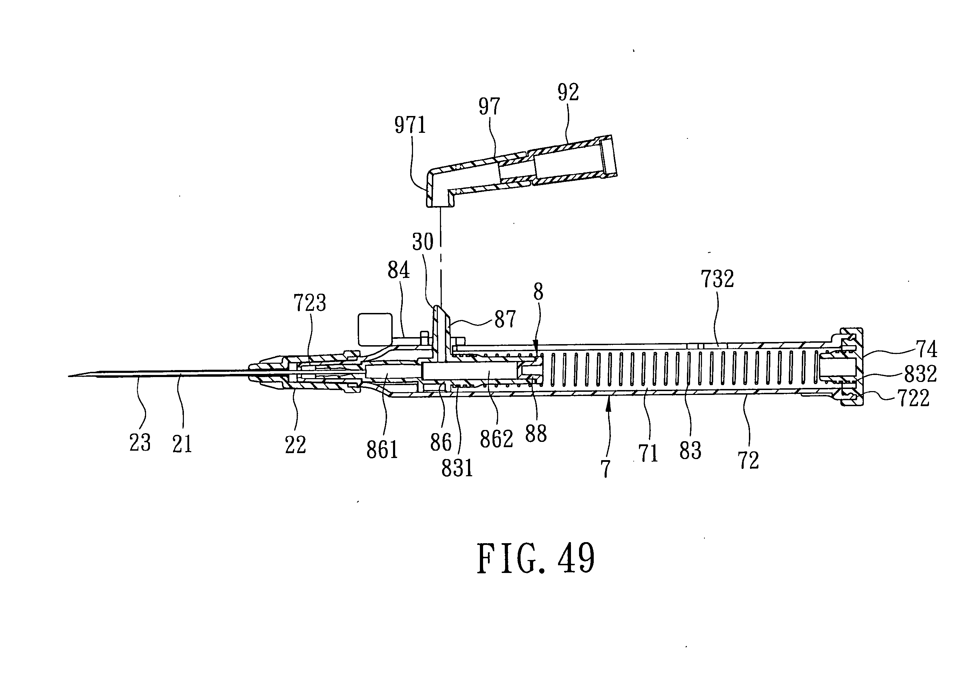

Referring to Figs. 48and49, thetwenty-seventhpreferred embodiment of an intravenous catheter introducing device according to this invention is shown to be similar to the twenty-sixth embodiment in construction. In this embodiment, a biasing spring 83 is received in the guiding groove 71 in the barrel 7, and has a front spring end 831 which is secured to the needle retracting member 8, and a rear spring end 832 which is secured to the tail end portion 722 of the barrel wall 72. The biasing spring 83 provides the same function as the biasing spring of the aforesaid second embodiment. Alternatively, the rear spring end 832 can be secured to the end cap 74, as shown in Fig. 49. In addition, a triggering member 84 is pivotally mounted on the barrel wall 72, and provides the same function as the triggering member of the aforesaid third embodiment.

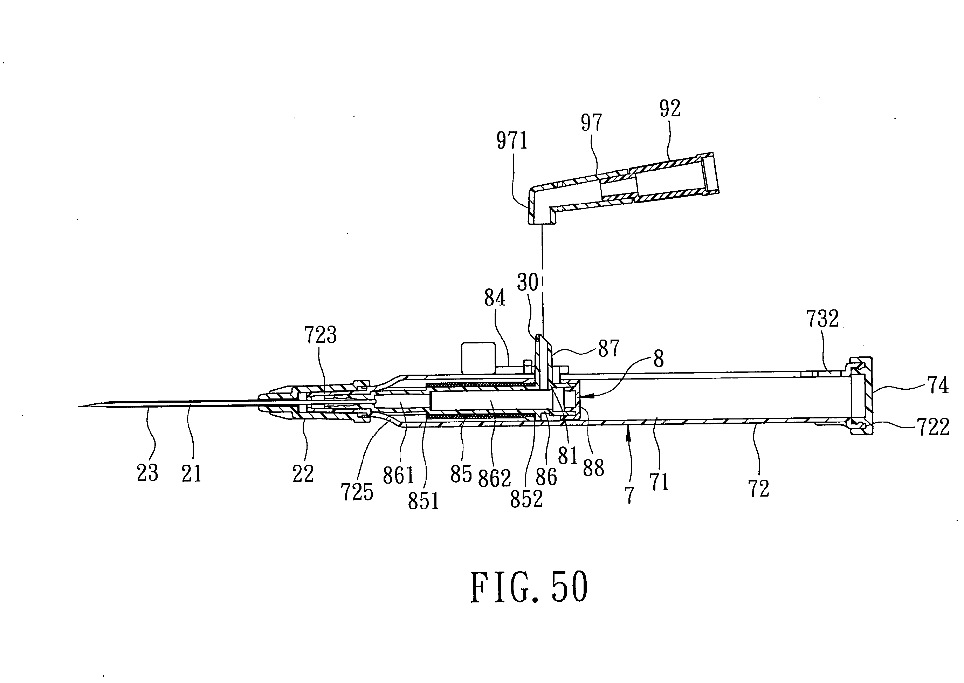

Referring to Fig. 50, the twenty-eighth preferred embodiment of an intravenous catheter introducing device according to this invention is shown to be similar to the twenty-seventh embodiment in construction. In this embodiment, a biasing spring 85 has front and rear spring ends 851,852 which abut against the shoulder portion 725 of the barrel 72 and the force effecting end 81, respectively, such that the biasing spring 85 is compressed by the needle retracting member 8 when the needle retracting member 8 is in the position of use. The biasing spring 85 provides the same function as the biasing spring of the aforesaid sixth embodiment.

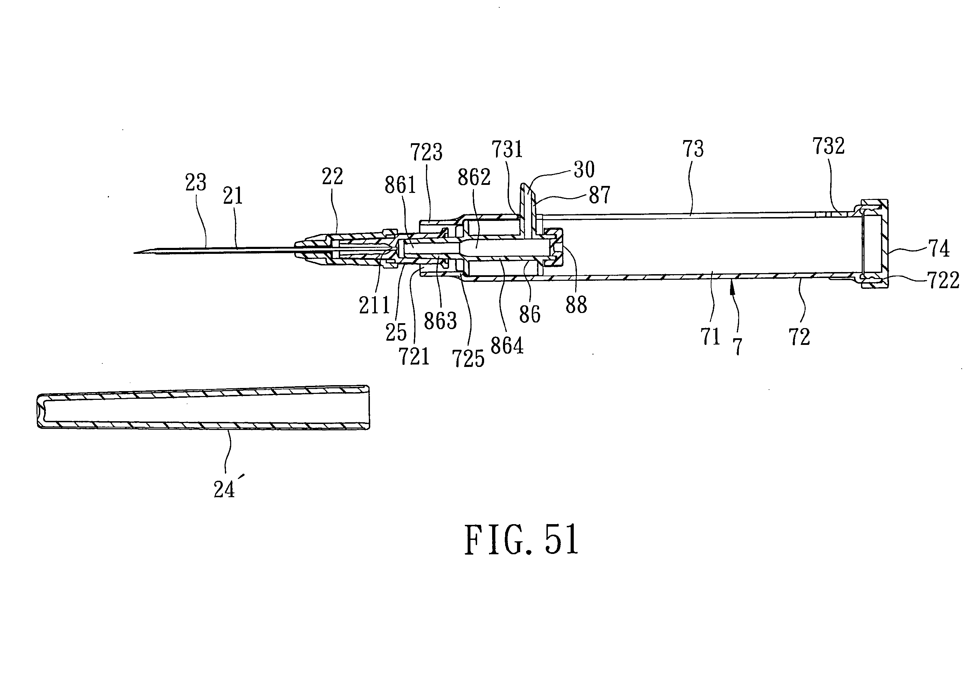

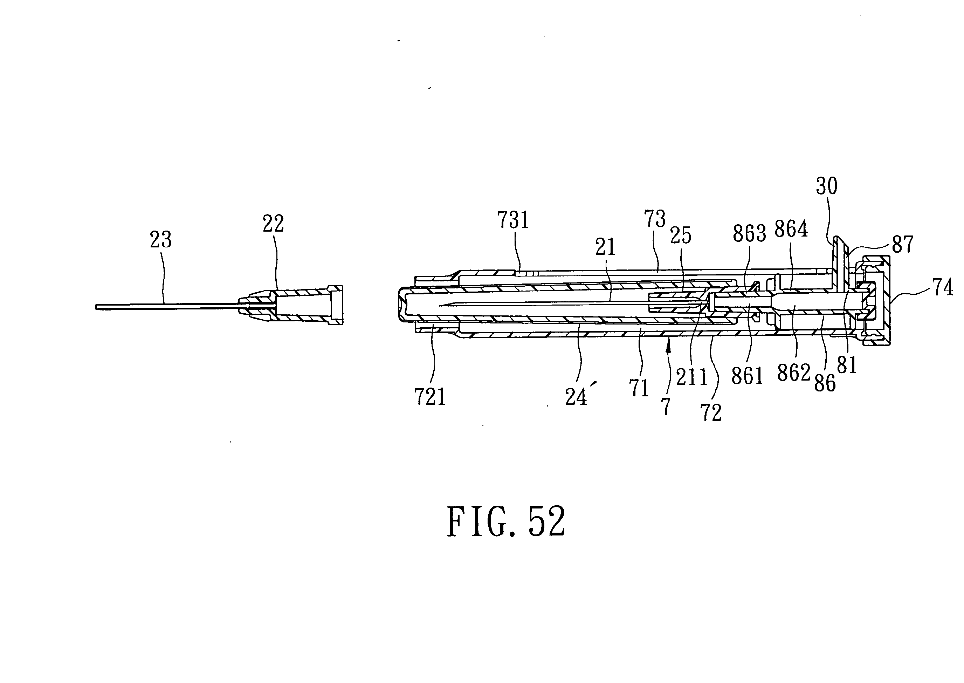

As shown in Figs. 51 and 52, the twenty-ninth preferred embodiment of an intravenous catheter introducing device according to this invention is shown to be similar to the twenty-third embodiment in construction. In this embodiment, the nose tubular end portion 723 of the barrel 7 has a relatively large inner-diameter such that the front wall portion 863 of the flashback member 86 can extend through the passage 721. In addition, a tubular needle seat 25 has a rear coupling portion which is detachably sleeved on the front wall portion 863 of the flashback member 86, and a front securing portion which is configured to secure the rear connecting end 211 of the needle cannula 21 so as to couple the front wall portion 863 with the rear connecting end 211, thereby placing the needle cannula 21 in fluid communication with the flashback chamber 861.

Moreover, a tip protector 24' is detachably sleeved on the catheter hub 22 for shielding the needle cannula 21 and the tubular catheter 23, and is configured to be detachably sleeved on the rear coupling portion of the tubular needle seat 25 when the catheter hub 22 is removed from the front securing portion for subsequent safe disposal when the needle retracting member 8 is displaced to the disposal position, as shown in Fig. 52.

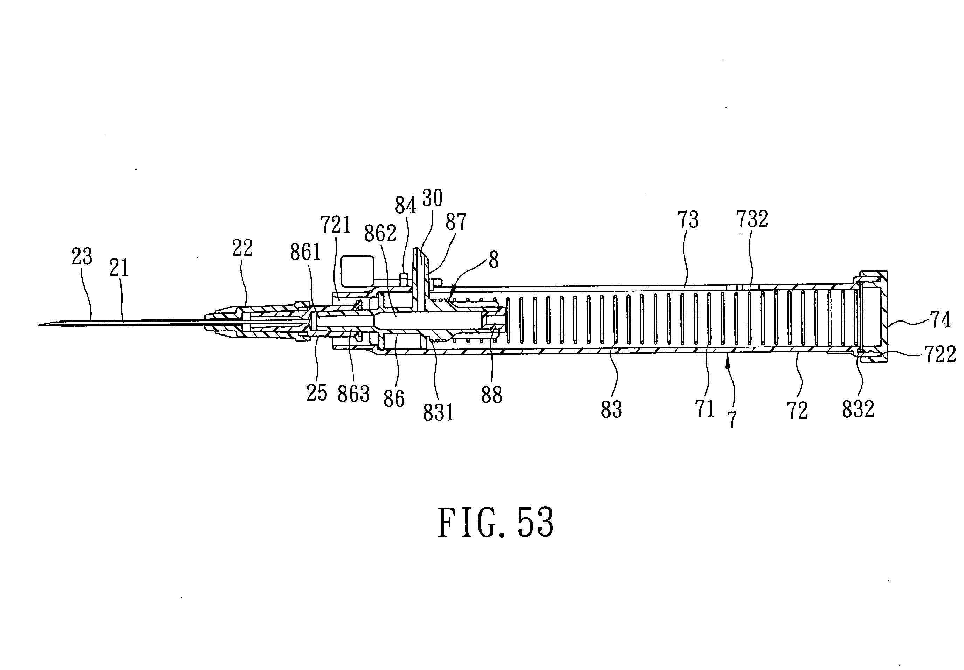

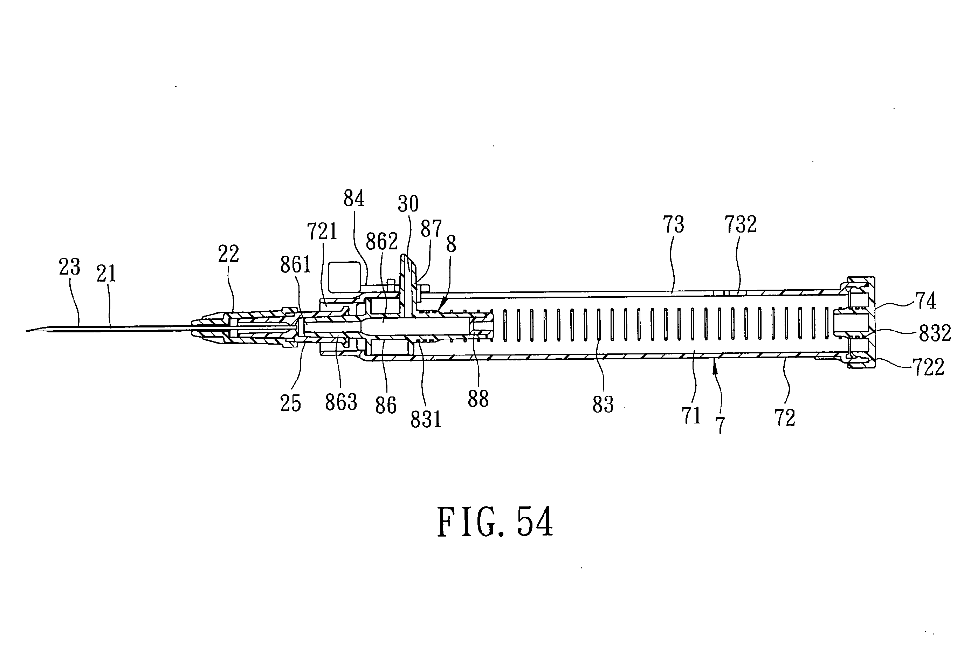

Referring to Figs. 53 and 54, the thirtieth preferred embodiment of an intravenous catheter introducing device according to this invention is shown to be similar to the twenty-ninth embodiment in construction. In this embodiment, a biasing spring 83 is received in the guiding groove 71 in the barrel 7, and has a front spring end 831 which is secured to the needle retracting member 8, and a rear spring end 832 which is secured to the tail end portion 722 of the barrel wall 72. The biasing spring 83 provides the same function as the biasing spring of the aforesaid second embodiment. Alternatively, the rear spring end 832 can be secured to the end cap 74, as shown in Fig. 54. In addition, a triggering member 84 is pivotally mounted on the barrel wall 72, and provides the same function as the triggering member of the aforesaid third embodiment.

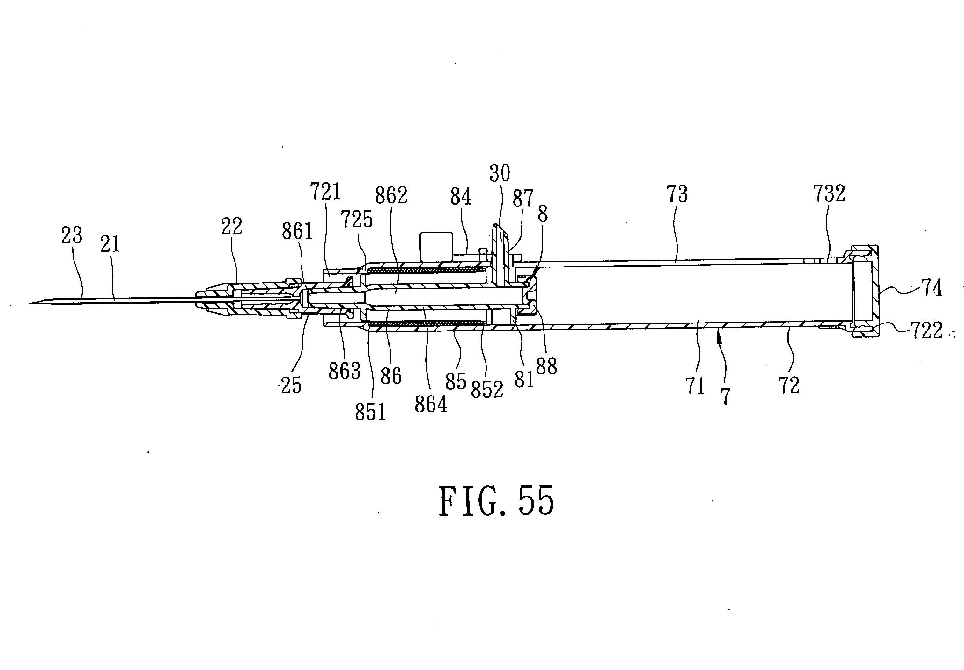

Referring to Fig. 55, the thirty-first preferred embodiment of an intravenous catheter introducing device according to this invention is shown to be similar to the thirtieth embodiment in construction. In this embodiment, a biasing spring 85 has front and rear spring ends 851,852 which abut against the shoulder portion 725 of the barrel 72 and the force effecting end 81, respectively, such that the biasing spring 85 is compressed by the needle retracting member 8 when the needle retracting member 8 is in the position of use. The biasing spring 85 provides the same function as the biasing spring of the aforesaid sixth embodiment.

It is noted that in each of the aforesaid twenty-seventh to thirty-first preferred embodiments, the tubular outlet member 30 can be removably connected to any one of the tubular hub 92, the tubular members 93,96, the end cover 95, and the flashback members 1,3,6,6' to meet clinical requirements.

## Claims

1. An intravenous catheter introducing device comprising:
a needle cannula (21) having a front segment (213) extending along an axis (X) to terminate at a tip end (212), and a rear connecting end (211) opposite to said front segment (213);
a catheter hub (22) which defines therein a duct (221) that permits extension of said front segment (213) of said needle cannula (21) therethrough; and
a tubular catheter (23) having a proximate segment which is inserted into said duct (221), and a distal segment which extends from said proximate segment to surround and sheathe said front segment (213) of said needle cannula (21) while permitting said tip end (212) to project forwardly of said distal segment for piercing a patient' s skin, **characterized by:**
a tubular outlet member (30) which is disposed downstream of said rear connecting end (211) of said needle cannula (21) in terms of inflow of a blood sample through said needle cannula (21) and which is radially offset from the axis (X) ;
a barrel (7) having a nose tubular end portion (723) which is detachably coupled with said duct (221) in the catheter hub (22), and which defines therein a passage (721) that is disposed to be communicated with said duct (221) and that is configured to permit said front segment (213) of said needle cannula (21) to pass therethrough, and a barrel wall (72) which extends from said nose tubular endportion (723) rearwardly along the axis (X) to terminate at a tail end portion (722) such that said tubular outlet member (30) is disposed outwardly of said barrel wall (72), and which defines a guiding groove (71) that is interposed between said nose tubular end portion (723) and said tail end portion (722), and that is communicated with said passage (721); and
a needle retracting member (8) which is disposed to be slidable relative to said barrel wall (72) toward said tail end portion (722), and which includes
a force effecting end (81) which is received in said guiding groove (71) to force said needle cannula (21) to move rearwards when said needle retracting member (8) is moved to slide toward said tail end portion (722), and
a shifter (87) configured to extend radially relative to the axis (X) and outwardly of said barrel wall (72) such that when said shifter (87) is caused to move by a user's fingertip, said needle retracting member (8) is moved to slide from a position of use, where said needle retracting member (8) is closer to said nose tubular end portion (723), to a disposal position, where said needle retracting member (8) is closer to said tail end portion (722) so as to withdraw said tip end (212) of said needle cannula (21) into said barrel wall (72).

2. The intravenous catheter introducing device of Claim 1, further **characterized by** a flashback member (1, 3, 6, 6' , 9) having front and rear ends (115,116) opposite to each other in a longitudinal direction, and a surrounding wall (12) interconnecting said front and rear ends (115,116), said surrounding wall (12) including
a rear wall portion (121,321) which is proximate to said rear end (116), and which cooperates with said rear end (116) to define a flashback chamber (11,31,61,61'), and
a front wall portion (122,322) which is proximate to said front end (115), and which is arranged such that said flashback chamber (1) is disposed downstream of said tubular outlet member (30).

3. The intravenous catheter introducing device of Claim 2, further **characterized by** a tubular bent member (27) which extends from said rear connecting end (211) of said needle cannula (21) to be fluidly communicated with said tubular outlet member (30).

4. The intravenous catheter introducing device of Claim 3, **characterized in that** said tubular bent member (27) is integrally formed with said needle cannula (21).

5. The intravenous catheter introducing device of Claim 3, **characterized in that** said tubular bent member (27) is in form of a flexible tube which is light transmissible to permit viewing of blood flowing therethrough.

6. The intravenous catheter introducing device of Claim 3, **characterized in that** said rear wall portion (121,321) of said flashback member (1,3,6,6') is configured such that volume of an accommodation space of said flashback chamber (11,31,61,61') is variable upon application of a manual force to said flashback chamber (11,31,61,61'), and such that, by varying the volume of said accommodation space with the manual force, a reduced pressure is created in said accommodation space to place said flashback chamber (11,31,61,61') in a ready position, where said tip end (212) is permitted to search for a vein of a patient for taking a blood sample after said tip end (212) has pierced into skin of the patient, while a biasing force acting on said flashback chamber (11,31,61,61') is generated as a result of a pressure difference between the reduced pressure and a pressure of an ambient atmosphere.

7. The intravenous catheter introducing device of Claim 6, further **characterized by** a pressure counteracting member (4) which is disposed to resist said biasing force so as to permit said flashback chamber (11,31,61,61') to be held in the ready position without the manual force once said tip end (212) has pierced into the skin of the patient in search of the vein.

8. The intravenous catheter introducing device of Claim 3, **characterized in that** said rear end (916) of said flashback member (9) is formed with a tubular port (911), said intravenous catheter introducing device further comprising a tubular hub (92) having a front hub segment (920) which is detachably inserted into said tubular port (911), a rear port segment (921) opposite to said front hub segment (920) in the longitudinal direction, and an air-permeable member (922) engaging and closing said rear port segment (921).

9. The intravenous catheter introducing device of Claim 3, **characterized in that** said flashback member (9) has a grip (912) which is disposed on said front wall portion for gripping by a user's finger.

10. The intravenous catheter introducing device of Claim 1, **characterized in that** said tubular outlet member (30) is integrally formed with said shifter (87) of said needle retracting member (8), said force effecting end (81) having an internal port (811) which is disposed downstream of said rear connecting end (211) of said needle cannula (21), said shifter (87) being configured to be a hollow stem so as to communicate said tubular outlet member (30) with said internal port (811).

11. The intravenous catheter introducing device of Claim 10, further **characterized by** a flashback member (86) having a surrounding wall that includes
a rear wall portion (864) which is disposed in said guiding groove (71) and which defines therein a passageway (862) that is disposed upstream of said internal port, and
a front wall portion (863) which is coupled with said rear connecting end (211) and which defines therein a flashback chamber (861) such that said flashback chamber (861) isdisposeddownstreamof said needle cannula (21).

12. The intravenous catheter introducing device of Claim 11, **characterized in that** said force effecting end (81) is integrally formed with said rear wall portion (864) of said flashback member (86) to permit said hollow stem to extend radially and outwardly of said barrel wall (72) .

13. The intravenous catheter introducing device of Claim 12, further **characterized by** a first tubular member (93) including a proximate tubular end which is detachably sleeved on and which surrounds said tubular outlet member (30), an extended portion which extends from said proximate tubular end in a direction substantially parallel to said tubular outlet member (30) to terminate at a distal tubular end (931), and an air-permeable member (932) disposed to engage said extended portion.

14. The intravenous catheter introducing device of Claim 13, further **characterized by** an end cap (94) which is hinged to said distal tubular end (931) of said first tubular member (93) to openably close said distal tubular end (931).

15. The intravenous catheter introducing device of Claim 12, **characterized in that** said tubular outlet member (30) has an upper region and a lower region which is configured to be shorter than said upper region so as to form an inclined end wall (301).

16. The intravenous catheter introducing device of Claim 15, further **characterized by** an end cover (95) which includes a tubular wall (951) that is detachably sleeved on and that surrounds said tubular outlet member (30) and that extends in a direction parallel to the tubular outlet member (30) to terminate at a tubular edge (952), and which has an end wall (953) connected and integrally formed with said tubular edge (952) such that said end wall (953) confronts said internal port (811); and
a sloping abutment (954) which extends from said end wall (953) and which is configured to mate with said inclined end wall (301) of said outlet tubular member (30) so as to establish a fluid-tight engagement between said tubular outlet member (30) and said end cover (95) .

17. The intravenous catheter introducing device of Claim 12, further **characterized by** a second tubular member (96) including a proximate tubular end (963) which is detachably sleeved on and which surrounds said tubular outlet member (30), an extended portion which extends from and which is bent relative to said proximate tubular end (963) in a direction substantially parallel to the axis (X) and which terminates at a distal tubular end (961), and an air-permeable member (962) engaging and closing said distal tubular end (961).

18. The intravenous catheter introducing device of Claim 12, further **characterized by**:
a third tubular member (97) including a proximate tubular end (971) which is detachably sleeved on and which surrounds said tubular outlet member (30), and an extended portion which extends from and which is bent relative to said proximate tubular end (971) in a direction substantially parallel to the axis (X) and which terminates at a distal tubular end (972); and
a tubular hub (92) having a front connecting end (920) which is detachably inserted into said distal tubular end (972), a rear connecting port (921) opposite to said front connecting end (920) in the longitudinal direction, and an air-permeable member (922) disposed to engage and close said distal tubular end (921).

19. The intravenous catheter introducing device of Claim 12, further **characterized by** a tubular needle seat (25) having a rear coupling portion which is detachably sleeved on said front wall portion (863) of said flashback member (86), and a front securing portion which is configured to secure said rear connecting end (211) of said needle cannula (21) so as to couple said front wall portion (863) with said rear connecting end (211), thereby placing said needle cannula (21) in fluid communication with said flashback chamber (861).

20. The intravenous catheter introducing device of Claim 19, further **characterized by** a tip protector (24) which is detachably sleeved on said catheter hub (22) for shielding said needle cannula (21) and said tubular catheter (23), and which is configured to be detachably sleeved on said rear coupling portion of said tubular needle seat (25) when said catheter hub (22) is removed from said front securing portion.

21. The intravenous catheter introducing device of Claim 1, **characterized in that** said barrel wall has an elongated guideway (73) which extends transversely therethrough to be communicated with said guiding groove (71) such that said shifter (87) is slidable along said guideway (73),
said device further comprising a bracing member (82) which has
an inner wall surface configured to partly surround said barrel wall about the axis (X) and to cross over said elongated guideway to terminate at two jaw regions that are disposed to abut against saidbarrel wall at two diametrically opposite areas, and
two rib barriers (822) that are configured to be inserted into said elongated guideway (73), and that cooperate with said jaw regions to guard against an undesired enlargement of said elongated guideway (73).

22. The intravenous catheter introducing device of Claim 1, further **characterized by** a protecting cap (26) which has a cap surrounding wall (262) that is detachably sleeved on said nose tubular end portion (723), and a cap end wall (261) which is disposed forwardly of said nose tubular end portion (723) so as to close said passage (721) when said needle cannula (21) is withdrawn into said barrel wall (72).

23. The intravenous catheter introducing device of Claim 1, **characterized in that** said barrel (7) has an elongated guideway (73) which extends transversely through said barrel wall (72) to be communicated with said guiding groove (71), and which has front and rear retaining regions (731, 732) that are proximate to and distal from said nose tubular end portion (723), respectively,
said shifter (87) being disposed in and being slidable along said guideway (73), and being retained in said front and rear retaining regions (731, 732), respectively, when said needle retracting member (8) is displaced to the position of use and the disposal position, respectively.

24. The intravenous catheter introducing device of Claim 23, further **characterized by** an end cap (74) which is fixedly disposed on said tail end portion (722) to close said guiding groove (71), and a biasing spring (83) which is received in said guiding groove (71) and which has a front spring end (831) that is secured to said force effecting end (81), and a rear spring end (832) that is secured to one of said tail end portion (722) and said end cap (74) such that said biasing spring (83) is tensioned by said needle retracting member (8) when said needle retracting member (8) is in the position of use.

25. The intravenous catheter introducing device of Claim 23, **characterized in that** said barrel wall (72) defines a shoulder portion (725) which is disposed proximate to said nose tubular end portion (723) and which faces said guiding groove (71), said device further comprising a biasing spring (85) which has front and rear spring ends (851,852) that abut against said shoulder portion (725) andsaidneedleretractingmember (8), respectively, such that said biasing spring (85) is compressed by said needle retracting member (8) when said needle retracting member (8) is in the position of use.

26. The intravenous catheter introducing device of Claim 23, further **characterized by** a gripped member (75) which is attached to said barrel wall (72) in the vicinity of said front retaining region (731) and which is configured to be held by a user's thumb for moving said shifter (87).

27. The intravenous catheter introducing device of Claim 23, further **characterized by** a triggering member (84) which is pivotally mounted on said barrel wall (72) at a fulcrum point, and which has a weight end (841) that is disposed to releasably hold said shifter (81) when said needle retracting member (8) is in the position of use, and a power end (842) that is disposed at an opposite side of said weight end (841) relative to said fulcrum point so as to be actuated to permit said weight end (841) to disengage from said shifter (81), thereby enabling said needle retracting member (8) to move to the disposal position.

28. The intravenous catheter introducing device of Claim 23, **characterized in that** said front retaining region (731) extends in a circumferential direction that surrounds the axis (X).

29. The intravenous catheter introducing device of Claim 1, further **characterized by** a tip protector (24,24') which is detachably sleeved on one of said barrel wall (72) (Fig. 7) and said catheter hub (22) (Fig.20,51) for shielding said needle cannula (21), said catheter hub (22) and said tubular catheter (23).
